(19) Europäisches Patentamt
European Patent Office
Office européen des brevets

(11) EP 3 898 889 B1

(12) **EUROPEAN PATENT SPECIFICATION**

(45) Date of publication and mention of the grant of the patent:
**11.09.2024 Bulletin 2024/37**

(21) Application number: **19836589.2**

(22) Date of filing: **20.12.2019**

(51) International Patent Classification (IPC):
***C09K 11/06*** *(2006.01)*

(52) Cooperative Patent Classification (CPC):
**C09K 11/06;** Y02E 10/549

(86) International application number:
**PCT/IB2019/061208**

(87) International publication number:
**WO 2020/129018 (25.06.2020 Gazette 2020/26)**

(54) **LUMINESCENT SOLAR CONCENTRATOR COMPRISING DITHIENYLPYRIDINETHIADIAZOLE COMPOUNDS**

LUMINESZENTER SOLARKONZENTRATOR MIT DITHIENYLPYRIDINTHIADIAZOLVERBINDUNGEN

CONCENTRATEUR SOLAIRE LUMINESCENT COMPRENANT DES COMPOSÉS DE TYPE DITHIÉNYLPYRIDINETHIADIAZOLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2018 IT 201800020419**

(43) Date of publication of application:
**27.10.2021 Bulletin 2021/43**

(73) Proprietor: **ENI S.p.A.**
**00144 Roma (IT)**

(72) Inventors:
- **ABBONDANZA, Luigi**
  **28100 Novara (IT)**
- **PROTO, Antonio Alfonso**
  **28100 Novara (IT)**
- **SCHIMPERNA, Giuliana**
  **28100 Novara (IT)**

(74) Representative: **Studio Torta S.p.A. et al**
**Via Viotti, 9**
**10121 Torino (IT)**

(56) References cited:
**WO-A1-2017/118464 US-A1- 2011 028 656**

- **CHO ARA ET AL: "Effects of alkyl side chain and electron-withdrawing group on benzo[1,2,5]thiadiazole-thiophene-based small molecules in organic photovoltaic cells", JOURNAL OF MATERIALS SCIENCE, KLUWER ACADEMIC PUBLISHERS, DORDRECHT, vol. 51, no. 14, 18 April 2016 (2016-04-18), pages 6770 - 6780, XP035914303, ISSN: 0022-2461, [retrieved on 20160418], DOI: 10.1007/S10853-016-9964-X**
- **GREGORY C. WELCH ET AL: "A modular molecular framework for utility in small-molecule solution-processed organic photovoltaic devices", JOURNAL OF MATERIALS CHEMISTRY, vol. 21, no. 34, 1 January 2011 (2011-01-01), GB, pages 12700, XP055621374, ISSN: 0959-9428, DOI: 10.1039/c1jm11963j**
- **WILLIAM R. MATEKER ET AL: "Molecular Packing and Arrangement Govern the Photo-Oxidative Stability of Organic Photovoltaic Materials", CHEMISTRY OF MATERIALS, vol. 27, no. 18, 2 September 2015 (2015-09-02), pages 6345 - 6353, XP055620738, ISSN: 0897-4756, DOI: 10.1021/acs.chemmater.5b02341**
- **YOHEI ADACHI ET AL: "Synthesis of organic photosensitizers containing dithienogermole and thiadiazolo[3,4-c]pyridine units for dye-sensitized solar cells", DALTON TRANSACTIONS, vol. 45, no. 35, 1 January 2016 (2016-01-01), pages 13817 - 13826, XP055620756, ISSN: 1477-9226, DOI: 10.1039/C6DT02469F**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 898 889 B1

- BATHULA CHINNA ET AL: "Microwave assisted synthesis of bithiophene based donor-acceptor-donor oligomers and their optoelectronic performances", JOURNAL OF MOLECULAR STRUCTURE, ELSEVIER, AMSTERDAM, NL, vol. 1139, 8 March 2017 (2017-03-08), pages 125 - 129, XP029966735, ISSN: 0022-2860, DOI: 10.1016/J.MOLSTRUC.2017.03.033
- TAO TANG ET AL: "Regiospecific protonation of organic chromophores", PHYSICAL CHEMISTRY CHEMICAL PHYSICS, vol. 18, no. 28, 1 January 2016 (2016-01-01), pages 18758 - 18766, XP055621370, ISSN: 1463-9076, DOI: 10.1039/C6CP02747D

## Description

CROSS-REFERENCE TO RELATED APPLICATIONS

This Patent Application claims priority from Italian Patent Application No. 102018000020419 filed on December 20, 2018.

TECHNICAL FIELD

The present invention relates to a luminescent solar concentrator (LSC) comprising at least one dithienylpyridinethiadiazole compound.

More particularly, the present invention relates to a luminescent solar concentrator (LSC) comprising at least one dithienylpyridinethiadiazole compound having the specific general formula (I) or (II) reported below.

The present invention also relates to the use of at least one dithienylpyridinethiadiazole compound having the specific general formula (I) or (II) reported below in the construction of luminescent solar concentrators (LSCs).

Said luminescent solar concentrator (LSC) can be advantageously used in the construction of photovoltaic devices (or solar devices) selected, for example, from photovoltaic cells (or solar cells), photovoltaic modules (or solar modules), both on rigid support, and on flexible support.

Accordingly, the present invention also relates to a photovoltaic device (or solar device) comprising at least one photovoltaic cell (or solar cell) and at least one luminescent solar concentrator (LSC) comprising at least one dithienylpyridinethiadiazole compound having the specific general formula (I) or (II) reported below.

It should be noted that some of the dithienylpyridinethiadiazole compounds having the specific general formula (I) or (II) are new. Consequently, said dithienylpyridinethiadiazole compounds having the specific general formula (I) or (II) are a further object of the present invention.

BACKGROUND ART

In the state of the art, one of the main limits to the exploitation of solar radiation energy is represented by the capability of photovoltaic devices (or solar devices) to optimally absorb only the radiations having wavelengths that fall within a narrow spectral range.

Against a spectral range of solar radiation that extends from wavelengths of about 300 nm to wavelengths of about 2500 nm, photovoltaic cells (or solar cells) based on crystalline silicon, for example, have an optimal absorption zone (effective spectrum) in the range 900 nm - 1100 nm, while polymeric photovoltaic cells (or solar cells) are liable to damage if exposed to radiation of wavelengths lower than about 500 nm, due to phenomena of induced photodegradation which become significant below this limit. Typically, the efficiency of the photovoltaic devices (or solar devices) of the state of the art is maximum in the region of the spectrum between 570 nm and 680 nm (yellow-orange).

The aforementioned drawbacks involve a limited external quantum efficiency (EQE) of photovoltaic devices (or solar devices), defined as the ratio between the number of electron-hole pairs generated in the semiconductor material of photovoltaic devices (or solar devices) and the number of incident photons on said photovoltaic devices (or solar devices).

To improve the external quantum efficiency (EQE) of photovoltaic devices (or solar devices) tools have been developed that, interposed between the source of light radiation (the sun) and photovoltaic devices (or solar devices), selectively absorb incident radiation having wavelengths outside the effective spectrum of said photovoltaic devices (or solar devices), emitting the absorbed energy in the form of photons of wavelength included in the effective spectrum. Said instruments are called luminescent solar concentrators (LSCs). When the energy of the photons emitted by the luminescent solar concentrators (LSCs) is higher than that of the incident photons, the photoluminescence process, including the absorption of solar radiation and the subsequent emission of photons at a shorter wavelength, is also called an up-conversion process. Conversely, when the energy of the photons emitted by luminescent solar concentrators (LSCs) is lower than that of incident photons, the photoluminescence process is called a "down-conversion" process (or "downshifting").

Generally, said luminescent solar concentrators (LSCs) consist of large sheets of a material transparent to solar radiation (for example, polymeric or inorganic glasses), inside which fluorescent compounds that act as spectrum converters, are dispersed, or chemically linked to said material. Due to the optical phenomenon of total reflection, the radiation emitted by the fluorescent compounds is "guided" towards the thin edges of the sheet where it is concentrated on photovoltaic cells (or solar cells) placed thereon. In this way, large surfaces of low-cost materials (photoluminescent sheets) can be used to concentrate light on small surfaces of high-cost materials [photovoltaic cells (or solar cells)].

The fluorescent compounds can be deposited on the glass support in the form of a thin film or, as in the case of polymeric materials, they can be dispersed inside the polymeric matrix. Alternatively, the polymeric matrix can be

directly functionalized with fluorescent chromophore groups.

Ideally, in order to be used in the construction of luminescent solar concentrators (LSCs), the fluorescent compounds must have the following characteristics:

- high luminescence quantum efficiency (Φ), said luminescence quantum efficiency (Φ) being defined according to the equation (1) below reported as the ratio between the number of photons emitted and the number of photons absorbed by a luminescent molecule per unit of time and has a maximum value of 1:

$$(\Phi) = \text{number of emitted photons/number of absorbed photons (1);}$$

- wide absorption band in the spectral region in which the photovoltaic device (or solar device) is poorly efficient;
- high absorption coefficient;
- narrow emission band in the spectral region in which the photovoltaic device (or solar device) is more efficient;
- well separated absorption and emission bands to avoid or minimize self-absorption phenomena.

It is known that some benzothiadiazole compounds, in particular 4,7-di-(thien-2'-yl)-2,1,3-benzothiadiazole (DTB), are fluorescent compounds usable in the construction of luminescent solar concentrators (LSCs). Compounds of this type have been described, for example, in the international patent application WO 2011/048458 in the name of the Applicant.

4,7-di-(thien-2'-yl)-2,1,3-benzothiadiazole (DTB) is characterized by an emission centred around 579 nm, which value corresponds to an energy well above the minimum threshold of operation of photovoltaic cells (or solar cells), which threshold, for example, corresponds to a wavelength of about 1100 nm for the most common silicon-based photovoltaic cells (or solar cells). Moreover, its absorption of the light radiation is intense and extended over a relatively wide range of wavelengths, including indicatively between 550 nm (the wavelength of the green radiation) and the ultraviolet. Finally, 4,7-di-(thien-2'-yl)-2,1,3-benzothiadiazole (DTB) has a Stokes' shift, in dichloromethane solution, equal to 134 nm, much higher than those of most of the commercial products so far proposed for use in luminescent solar concentrators (LSCs).

For these reasons, the use of 4,7-di-(thien-2'-yl)-2,1,3-benzothiadiazole (DTB) has made it possible to create luminescent solar concentrators (LSCs) of excellent quality.

However, 4,7-di-(thien-2'-yl)-2,1,3-benzothiadiazole (DTB), although it absorbs a significant part of the solar spectrum, shows a modest absorption in its regions of greater wavelength, corresponding to the yellow and red radiations which, therefore, cannot be converted into others which are more effectively exploited by the photovoltaic cell (or solar cell).

Dithienylpyridinethiadiazole compounds are also known.

For example, Bathula C. and others, in "Journal of Fluorescence" (2016), Vol. 26, Issue 3, p. 1045-1052, report 4,7-bis[(4- (2-ethylhexyl) -thiophene-2-yl]-[1,2,5]thiadiazole[3,4-c]pyridine having the following formula (A):

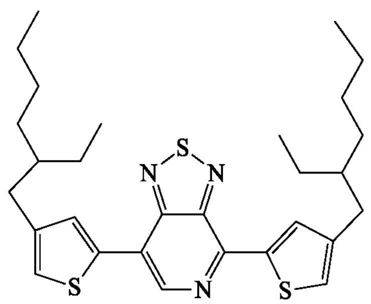

(A)

as comonomer for the synthesis of thiadiazole[3,4-c]pyridine based copolymers usable in the field of organic electronics, for example in organic field-effect transistors ("Organic Emitting Diodes" - OLEDs).

Welch G. C. and others, in "Journal of Materials Chemistry" (2011), Vol. 21, p. 12700-12709, report organic molecules comprising thiadiazole[3,4-c]pyridine, including the compound having formula (6):

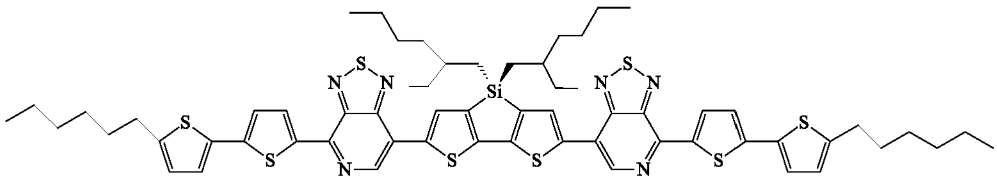

(6)

and their use as electron donor materials in organic photovoltaic cells having architecture known as bulk heterojunction obtained through processes in solution. Said organic molecules comprising thiadiazole[3,4-c]pyridine, including the compound having formula (6), as well as their use as electron donor materials in organic photovoltaic cells having architecture known as bulk heterojunction obtained by means of processes in solution, are also described in US patent application US 2014/0167002.

Bazan G. C. and others in "Journal of the American Chemical Society" (2013), Vol. 135(6), p. 2298-2305, report the synthesis of two organic molecules including thiadiazole[3,4-c]pyridine, i.e. the compound having formula (B) and the compound having formula (C):

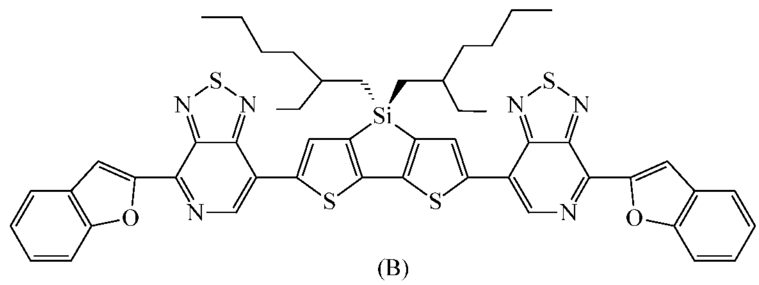

(B)

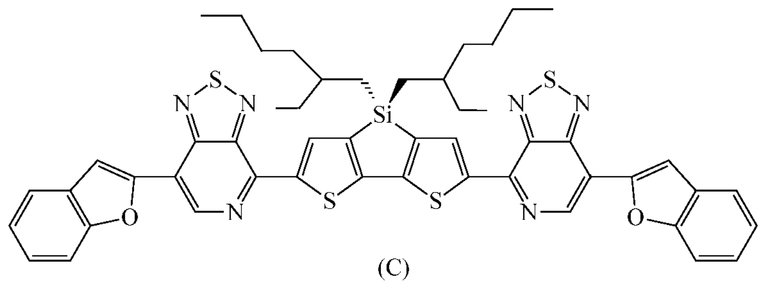

(C)

usable in the field of organic electronics.

Other documents discloses dithienylpyridinethiadiazole compounds which are integrated directly into organic-based photovoltaic cells or other organic electronic devices which carry out photon-to-current conversion:

ARA C. et al., "Effects of alkyl side chain and electron-withdrawing group on benzo[1,2,5]thiadiazole-thiophene-based small molecules in organic photovoltaic cells", Journal Of Materials Science, Kluwer Academic Publishers, Dordrecht, Vol. 51, No. 14 (2016-04-18), pages 6770 - 6780;

US2011028656;

MATEKER W. et al., "Molecular Packing and Arrangement Govern the Photo-Oxidative Stability of Organic Photovoltaic Materials", Chemistry Of Materials, Vol. 27, No. 18 (2015-09-02), pages 6345 - 6353;

ADACHI Y. et al., "Synthesis of organic photosensitizers containing dithienogermole and thiadiazolo[3,4-c]pyridine units for dye-sensitized solar cells", Dalton Transactions, Vol. 45, No. 35 (2016-01-01), pages 13817 - 13826;

CHINNA B. et al., "Microwave assisted synthesis of bithiophene based donor-acceptor-donor oligomers and their optoelectronic performances", Journal Of Molecular Structure, Elsevier, Amsterdam, NL, Vol. 1139 (2017-03-08), pages 125 - 129;

TANG T. et al., "Regiospecific protonation of organic chromophores", Physical Chemistry Chemical Physics, Vol. 18, No. 28 (2016-01-01), pages 18758 - 18766.

WO2017118464A discloses mixtures which have liquid-crystalline properties and can be used in devices which regulate the transmission of light through an area element. In some embodiments, the mixtures include dyes selected from specific benzothiadiazoles.

The Applicant has therefore posed the problem of finding compounds able to have a good absorption in the regions of the solar spectrum with a longer wavelength, corresponding to the yellow and red radiations and, consequently, to give comparable or even higher performances, in particular in terms of the power generated by the photovoltaic devices (or solar devices) in which they are used, with respect to the known benzothiadiazole compounds, in particular compared to the 4,7-di-(thien-2'-il)-2,1,3-benzothiadiazole (DTB).

DISCLOSURE OF INVENTION

The Applicant has now found dithienylpyridinethiadiazole compounds having a specific general formula [i.e having general formula (I) or (II) reported below], which can be advantageously used in the construction of luminescent solar concentrators (LSCs). Said luminescent solar concentrators (LSCs) can, in turn, be advantageously used together, for example, with photovoltaic cells (or solar cells), in the construction of photovoltaic devices (or solar devices). Said dithienylpyridinethiadiazole compounds are capable of giving comparable or even higher performance, in particular in terms of the power generated by the photovoltaic devices in which they are used, compared to the known benzothiadiazole compounds, in particular with respect to 4,7-di-(thien-2'-yl)-2,1,3-benzothiadiazole (DTB).

An object of the present invention is therefore a luminescent solar concentrator (LSC) as claimed by claim 1, comprising at least one dithienylpyridinethiadiazole compound having general formula (I) or (II):

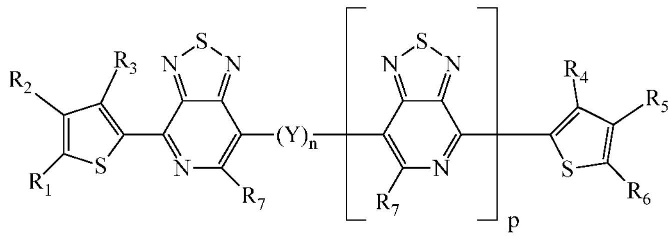

(I)

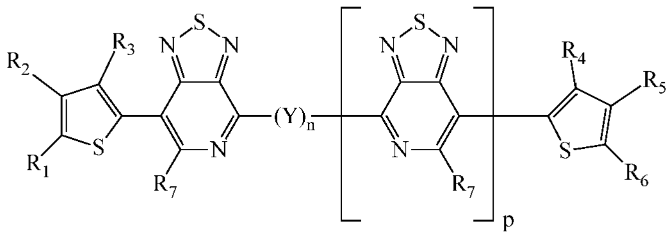

(II)

wherein:

- $R_1$, $R_2$, $R_3$, $R_4$, $R_5$ and $R_6$, mutually identical or different, represent a hydrogen atom; or they are selected from linear or branched, saturated or unsaturated $C_1$-$C_{20}$, preferably $C_1$-$C_8$, alkyl groups, optionally containing heteroatoms, optionally substituted aryl groups, optionally substituted heteroaryl groups;
- $R_7$, mutually identical or different, represent a hydrogen atom; or they are selected from linear or branched, saturated or unsaturated $C_1$-$C_{20}$, preferably $C_1$-$C_8$, alkyl groups, optionally containing heteroatoms, optionally substituted aryl groups, optionally substituted heteroaryl groups, optionally substituted phenoxy groups;
- or $R_1$ and $R_2$ or $R_2$ and $R_3$ and/or $R_4$ and $R_5$ or $R_5$ and $R_6$, can be optionally linked together so as to form, together with the carbon atoms to which they are linked, a saturated, unsaturated, or aromatic, cycle or polycyclic system containing from 3 to 14 carbon atoms, preferably from 4 to 6 carbon atoms, optionally containing one or more heteroatoms such as, for example, oxygen, sulfur, nitrogen, silicon, phosphorus, selenium;
- Y represents a divalent cycle or polycyclic group containing one or more aromatic or heteroaromatic rings, said aromatic or heteroaromatic rings being optionally substituted;
- n is 0 or 1;
- p is 0 or 1.

As stated above, some of the dithienylpyridinethiadiazole compounds having the specific general formula (I) or (II) are new.

It is therefore a further object of the present invention, a dithienylpyridinethiadiazole compound ad claimed by claim 5.

For the purpose of the present description and of the following claims, the definitions of the numerical intervals

always include the extreme values unless otherwise specified.

For the purpose of the present description and of the following claims, the term "comprising" also includes the terms "which essentially consists of" or "which consists of".

For the purpose of the present description and of the following claims, the term "$C_1$-$C_{20}$ alkyl groups" means alkyl groups having from 1 to 20 carbon atoms, linear or branched, saturated or unsaturated. Specific examples of $C_1$-$C_{20}$ alkyl groups are: methyl, ethyl, n-propyl, iso-propyl, n-butyl, iso-butyl, *tert*-butyl, pentyl, 2-ethyl-hexyl, hexyl, heptyl, octyl, nonyl, decile, dodecile.

For the purpose of the present description and of the following claims, the term "$C_1$-$C_{20}$ alkyl groups optionally containing heteroatoms" means alkyl groups having from 1 to 20 carbon atoms, linear or branched, saturated or unsaturated, in which at least one of the hydrogen atoms are substituted with a heteroatom selected from: halogens such as, for example, fluorine, chlorine, preferably fluorine; nitrogen; sulfur; oxygen. Specific examples of $C_1$-$C_{20}$ alkyl groups optionally containing heteroatoms are: fluoromethyl, difluoromethyl, trifluoromethyl, trichloromethyl, 2,2,2-trifluoroethyl, 2,2,2-trichlororoethyl, 2,2,3,3-tetrafluoropropyl, 2,2,3,3,3-pentafluoropropyl, perfluoropentyl, perfluoroctyl, perfluorodecyl, oxymethyl, oxyethyl, oxybutylthiomethyl, thioethyl, dimethylamino, propylamino, dioctylamino, methylthioether, ethylthioether, butylthioether.

For the purpose of the present description and of the following claims, the term "aryl groups" means aromatic carbocyclic groups. Said aryl groups can optionally be substituted with one or more groups, mutually identical or different, selected from: halogen atoms such as, for example, fluorine, chlorine, preferably fluorine; hydroxyl groups; $C_1$-$C_{20}$ alkyl groups; $C_1$-$C_{20}$ alkoxy groups; cyan groups; amino groups; nitro groups; aryl groups; phenoxy groups; ester groups; thioether groups. Specific examples of aryl groups are: phenyl, methylphenyl, dimethylphenyl, trimethylphenyl, di-isopropylphenyl, *tert*-butylphenyl, methoxyphenyl, hydroxyphenyl, phenyloxyphenyl, fluorophenyl, pentafluorophenyl, chlorophenyl, nitrophenyl, diphenylamino, dimethylaminophenyl, diphenylaminophenyl, naphthyl, phenylnaphthyl, phenanthrene, anthracene, phenylthioether, benzylthioether, 2-phenoxyphenyl, 4-phenoxyphenyl, 2,4-diphenoxyphenyl, 2,6-diphenoxyphenyl, 2,4,6-triphenoxyphenyl.

For the purpose of the present description and of the following claims, the term "heteroaryl groups" means heterocyclic aromatic, penta- or hexa-atomic groups, also benzocondensed or heterobicyclic, containing from 4 to 60 carbon atoms and from 1 to 4 heteroatoms selected from nitrogen, oxygen, sulfur, silicon, selenium, phosphorus. Said heteroaryl groups can be optionally substituted with one or more groups, mutually identical or different, selected from: halogen atoms such as, for example, fluorine, chlorine, bromine, preferably fluorine; hydroxyl groups; $C_1$-$C_{12}$ alkyl groups; $C_1$-$C_{12}$ alkoxy groups; $C_1$-$C_{12}$ thioalkoxy groups; $C_3$-$C_{24}$ tri-alkylsilyl groups; $C_3$-$C_{24}$ tri-alkoxysilyl groups; polyethyleneoxy groups; cyano groups; amino groups; $C_1$-$C_{12}$ mono- or di-alkylamine groups; nitro groups. Specific examples of heteroaryl groups are: pyridine, methylpyridine, methoxypyridine, phenylpyridine, fluoropyridine, pyrimidine, pyridazine, pyrazine, triazine, tetrazine, quinoline, quinoxaline, quinazoline, furan, thiophene, hexylthiophene, bromothiophene, dibromothiophene, pyrrole, oxazole, thiazole, isothiazole, oxadiazole, thiadiazole, pyrazole, imidazole, triazole, tetrazole, indole, benzofuran, benzothiophene, benzooxazole, benzothiazole, benzooxadiazole, benzothiadiazole, benzopyrazole, benzimidazole, benzotriazole, triazolepyridine, coumarin.

For the purpose of the present description and of the following claims, the term "optionally substituted phenoxy groups" means $C_6H_5O$ phenoxy groups, optionally substituted with one or more groups, mutually identical or different, selected from: halogen atoms such as, for example, fluorine, chlorine, preferably fluorine; $C_1$-$C_{20}$ alkyl groups; $C_1$-$C_{20}$ alkoxy groups; cyano groups; amino groups; nitro groups. Specific examples of $C_6H_5O$ phenoxy groups are: phenoxy, 4-nitro-phenoxy, 2,4-di-nitrophenoxy, 2-chloro-4-nitrophenoxy, 2-fluoro-4-nitrophenoxy, 3-fluoro-4-nitrophenoxy, 5-fluoro-2-nitrophenoxy, 2-aminophenoxy.

For the purpose of the present description and of the following claims, the term "cycle or polycyclic system" means a system containing one or more rings containing from 3 to 14 carbon atoms, saturated or unsaturated, optionally containing heteroatoms selected from nitrogen, oxygen, sulfur, silicon, selenium, phosphorus. Specific examples of cycle or polycyclic system are: thieno[3,2-b]thiophene, thiadiazole, benzothiophene, quinoxaline, pyridine.

For the purpose of the present description and of the following claims, the term "divalent cycle or polycyclic group containing one or more aromatic or heteroaromatic rings" means groups containing an optionally substituted aromatic ring or an optionally substituted heteroaromatic ring, or groups containing more optionally substituted aromatic rings or more optionally substituted heteroaromatic rings, said aromatic or heteroaromatic rings being condensed or linked together by simple bonds or by ligand groups. Specific examples of cycle or polycyclic groups containing one or more aromatic or heteroaromatic rings are: thiophene, pyrrole, furan, phosphol, benzodithiophene, spirofluorene, spirothiophene, bitiophene, tert-thiophene, thienothiophene, dithienotiophene, benzothiophene, iso-benzothiophene, benzodithiophene, cyclopentadithiophene, silacyclopentadiene, silacyclopentadienebithiophene, indole, benzene, naphthalene, anthracene, perylene, indene, fluorene, pyrene, azulene, pyridine, oxazole, thiazole, thiazine, pyrimidine, pyrazine, imidazole, benzoxazole, benzooxadiazole, benzothiazole, benzimidazole, benzofuran, isobenzofuran, thiadiazole, dithienopyrrole, dithiophosphol, dithienothiophene, thieno[3,2-b]thiophene, carbazole-9,9-RR'-9H-fluorene, 9-R-9H-carbazole, 3,3'-RR'-silylene-2,2'-bitiophene, 3,3'-RR'-cyclopenta[2,1-b:3,4-b']-dithiophene in which R and R', mutually identical or different, represent a linear or branched $C_1$-$C_{30}$ alkyl group or a $C_6$-$C_{30}$ aryl group.

According to a preferred embodiment of the present invention, in said general formula (I):

- $R_1$, $R_2$, $R_3$, $R_4$, $R_5$, $R_6$ e $R_7$, mutually identical, represent a hydrogen atom; n and p are 0; or
- $R_2$, $R_3$, $R_4$, $R_5$ and $R_7$, mutually identical, represent a hydrogen atom; $R_1$ and $R_6$, mutually identical, are selected from optionally substituted aryl groups, preferably they are a 2,5-dimethylphenyl group; n and p are 0; or
- $R_2$, $R_3$, $R_4$, $R_5$ and $R_7$, mutually identical, represent a hydrogen atom; $R_1$ and $R_6$, mutually identical, are selected from optionally substituted aryl groups, preferably they are a 2,6-dimethylphenyl group; n and p are 0; or
- $R_2$, $R_3$, $R_4$, $R_5$ and $R_7$, mutually identical, represent a hydrogen atom; $R_1$ and $R_6$, mutually identical, are selected from optionally substituted aryl groups, preferably they are a 2-phenoxyphenyl group; n and p are 0; or
- $R_2$, $R_3$, $R_4$, $R_5$, $R_6$ and $R_7$, mutually identical, represent a hydrogen atom; $R_1$ is selected from optionally substituted aryl groups, preferably it is a 2-phenoxyphenyl group; n and p are 0; or
- $R_2$, $R_3$, $R_4$, $R_5$ and $R_7$, mutually identical, represent a hydrogen atom; $R_1$ and $R_6$, are selected from optionally substituted aryl groups, preferably they are a 2,4,6-triphenoxyphenyl group; n and p are 0.

Specific examples of dithienylpyridinethiadiazole compounds having general formula (I) useful for the purpose of the present invention are reported in Table 1.

Table 1

| | |
|---|---|
| 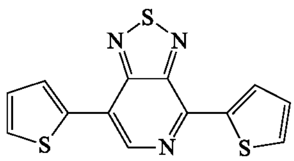 (DTP) | 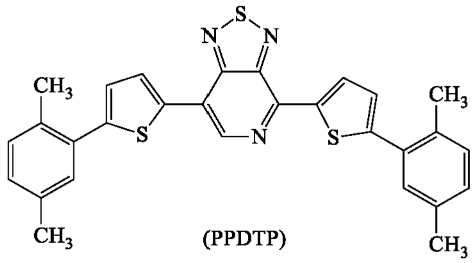 (PPDTP) |
| 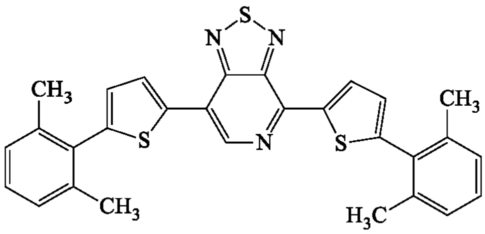 (MPDTP) | 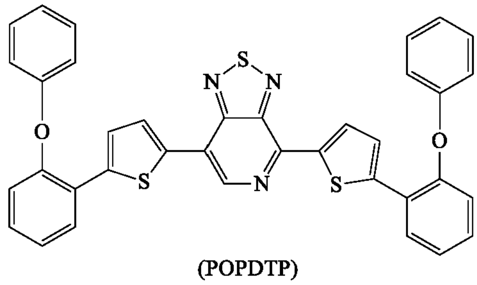 (POPDTP) |
| 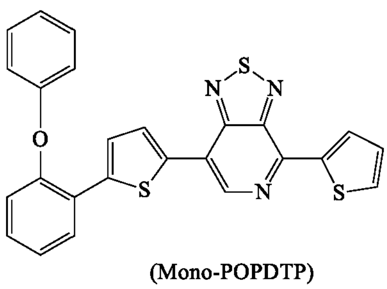 (Mono-POPDTP) | 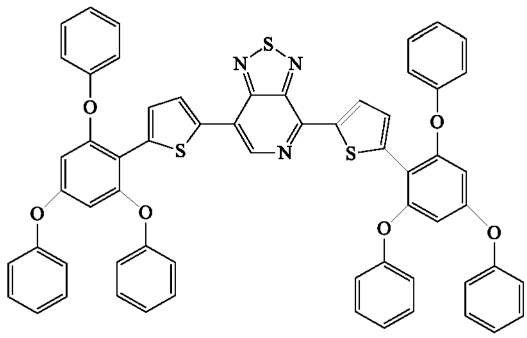 (Tri-POPDTP) |

The dithienylpyridinethiadiazole compound having general formula (I) or (II) can be obtained according to processes known in the art. For example, said dithienylpyridinethiadiazole compound having general formula (I) or (II) can be obtained by operating as described, for example, by Leclerc M. and others, in "Journal of the American Chemical Society" (2008), Vol. 130(2), p. 732-742; or by Jen A. K.-Y. in "Journal of Materials Chemistry" (2011), Vol. 21, p. 13247-13255; or by Welch G. C. and others, in "Journal of the American Chemical Society" (2011), Vol. 133(12), p.

4632-4644; or in the Italian patent application MI2018000000667 in the name of the Applicant and incorporated herein by reference. Further details relating to the processes for preparing said dithienylpyridinethiadiazole compound having general formula (I) or (II) can be found in the following examples.

A further object of the present invention is the use of at least one dithienylpyridinethiadiazole compound having general formula (I) or (II) in the construction of luminescent solar concentrators (LSCs) as claimed by claim 3.

The dithienylpyridinethiadiazole compound having general formula (I) or (II) can be used in said luminescent solar concentrator (LSC) in the following forms: dispersed in the polymer or in the glass, chemically linked to the polymer or glass, in solution, in the form of a gel.

For example, the luminescent solar concentrator (LSC) can contain a transparent matrix, where the term transparent matrix means any transparent material used in the form of a support, a binder, or a material in which at least one dithienylpyridinethiadiazole compound having general formula (I) or (II) is dispersed or incorporated. The material used for the matrix is transparent, as such, to the radiations of interest and, in particular, to the radiations having a frequency comprised in the effective spectrum of the photovoltaic device (or solar device) such as, for example, the photovoltaic cell (or solar cell) in which it is used. Materials suitable for the purpose of the present invention can therefore be selected from transparent materials at least at radiations having a wavelength ranging from 250 nm to 1100 nm.

The transparent matrix which can be used for the purpose of the present invention can be selected, for example, from polymeric materials or glassy materials. Said matrix is characterized by a high transparency and a high duration in relation to heat and light. Polymeric materials which can be advantageously used for the purpose of the present invention are, for example, polymethylmethacrylate (PMMA), epoxy resins, silicone resins, polyalkylene terephthalates, polycarbonates, polystyrene, polypropylene. Glassy materials that can be advantageously used for the purpose of the present invention are, for example, silicas.

In the case in which the matrix is of the polymeric type, said at least one dithienylpyridinethiadiazole compound having general formula (I) or (II) can be dispersed in the polymer of said matrix by, for example, melt dispersion, and subsequent formation of a sheet comprising said polymer and said at least one dithienylpyridinethiadiazole compound having general formula (I) or (II), operating, for example, according to the so-called "casting" technique. Alternatively, said at least one dithienylpyridinethiadiazole compound having general formula (I) or (II) and the polymer of said matrix can be solubilized in at least one solvent obtaining a solution which is deposited on a sheet of said polymer, forming a film comprising said at least one dithienylpyridinethiadiazole compound having general formula (I) or (II) and said polymer, operating, for example, by using a "Doctor Blade" film applicator: subsequently, said solvent is allowed to evaporate.

In the case in which the matrix is of the glassy type, said at least one dithienylpyridinethiadiazole compound having general formula (I) or (II) can be solubilized in at least one solvent obtaining a solution which is deposited on a sheet of said matrix of the glassy type, forming a film comprising said at least one dithienylpyridinethiadiazole compound having general formula (I) or (II), operating, for example, by using a "Doctor Blade" film applicator: subsequently said solvent is allowed to evaporate.

A further object of the present invention is a photovoltaic device (or solar device) comprising at least one photovoltaic cell (or solar cell), and at least one luminescent solar concentrator (LSC) comprising at least one dithienylpyridinethiadiazole compound having formula general (I) or (II), as claimed by claim 4.

Said photovoltaic device (or solar device) can be obtained, for example, by assembling the aforesaid luminescent solar concentrator with at least one photovoltaic cell (or solar cell).

In accordance with a preferred embodiment of the present invention, the aforesaid solar concentrator can be made in the form of a transparent sheet obtained by solubilization of said at least one dithienylpyridinethiadiazole compound having general formula (I) or (II) and of the polymer of the matrix of the polymeric type in at least one solvent obtaining a solution which is deposited on a sheet of said polymer, forming a film comprising said at least one dithienylpyridinethiadiazole compound having general formula (I) or (II) and said polymer, operating, for example, by using of a "Doctor Blade" film applicator: subsequently, said solvent is allowed to evaporate. In said photovoltaic devices (or solar devices), said sheets can then be coupled to a photovoltaic cell (or solar cell).

## BEST MODE FOR CARRYING OUT THE INVENTION

In order to better understand the present invention and to put it into practice, some illustrative and non-limiting examples thereof are reported below.

### EXAMPLE 1

### Synthesis of 4,7-dithienyl[1,2,51thiadiazole[3,4-c]pyridine (DTP)

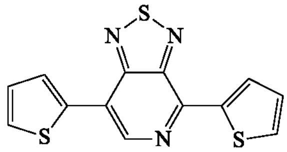

(DTP)

In a 50 ml microwave tube, under argon flow, dibromopyridinethiadiazole (Aldrich) (0.37 g; 1.25 mmoles), dimethylformamide (DMF) (Aldrich) (14 ml), 2-tributylstannylthiophene (Aldrich) (0.97 g; 825 μl; 2.6 mmoles) and tetrakis(triphenylphosphine)palladium(0) (Aldrich) (0.008 g; $6.9 \times 10^{-3}$ mmoles) are charged: the tube was kept, under argon flow, under stirring, for 30 seconds, at room temperature (25°C). The tube was then sealed and heated, under stirring, in a microwave setting the temperature ramp as follows: from room temperature (25°C) to 120°C in 2 minutes; 2 minutes at 120°C; from 120°C to 140°C in 2 minutes, 2 minutes at 140°C; from 140°C to 170°C in 2 minutes; 36 minutes at 170°C. Subsequently, the whole was poured into distilled water (40 ml) and extracted with dichloromethane (Aldrich) (3 × 20 ml): the organic phase obtained was washed to neutrality with distilled water (3 × 25 ml) and subsequently dried on sodium sulfate (Aldrich). After having removed most of the residual solvent by distillation under reduced pressure, the obtained residue was added, by dripping, to 50 ml of methanol (Aldrich), obtaining a precipitate which was recovered by filtration and subsequently purified by elution on chromatography column of silica gel [eluent: mixture of n-heptane (Aldrich)/dichloromethane (Aldrich) in a ratio of 9/1 (v/v)] obtaining 0.21 g of 4,7-dithienyl[1,2,5]thiadiazole[3,4-c]pyridine (DTP) (yield = 92%).

EXAMPLE 2

Synthesis of 4,7-di(5-(1-(2,5-dimethyl)phenyl)thiophen-2-yl)-[1,2,5]thiadiazole[3,4-c]pyridine (PPDTP)

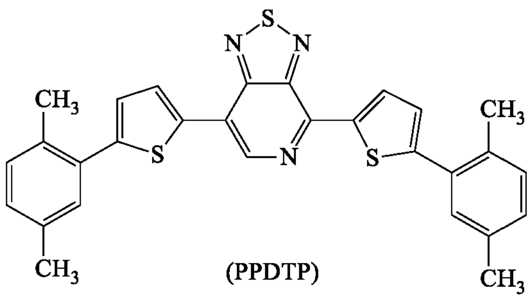

(PPDTP)

(1) Synthesis of 2-(2,5-dimethylphenyl)thiophene (a)

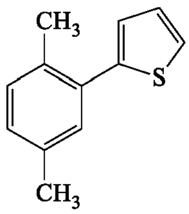

(a)

In a 100 ml flask, equipped with magnetic stirring, thermometer and coolant, in an inert atmosphere, 2,5-dimethylphenylboronic acid (Aldrich) (1.1 g; 7.3 mmoles) and potassium carbonate (Aldrich) (3.4 g; 24.6 mmoles) dissolved in distilled water (10 ml) were added to a solution of 2-bromothiophene (Aldrich) (1 g; 6.13 mmoles) in dioxane (Aldrich) (30 ml). After removing the air present by 3 vacuum/nitrogen cycles, tetrakis(triphenylphosphine)-palladium(0) (Aldrich) (0.14 g; 0.12 mmoles) was added obtaining a reaction mixture which was immersed into a bath pre-heated to

85°C and kept, under stirring, at said temperature, for 16 hours. Subsequently, the obtained reaction mixture, after addition of a saturated aqueous solution of sodium chloride (Aldrich) (50 ml), was extracted with ethyl ether (Aldrich) (3 × 20 ml): the obtained organic phase was washed to neutrality with distilled water (3 × 25 ml) and subsequently dried on sodium sulfate (Aldrich). After removing the solvent by distillation under reduced pressure, the obtained residue was purified by elution on a chromatographic column of silica gel [eluent: n-heptane(Aldrich)] obtaining 1 g of 2-(2,5-dimethylphenyl)thiophene (a) (yield = 87%).

(2) Synthesis of 4,7-di(5-(1-(2,5-dimethyl)phenyl)thiophen-2-yl)-[1,2,5]-thiadiazole[3,4-c]pyridine (PPDTP)

In a 100 ml flask equipped with magnetic stirring, thermometer and coolant, in an inert atmosphere, n-butyllithium (Aldrich) [1.6 M solution in hexane (Aldrich)] (2.5 ml; 4 mmoles) was added to a solution of 2-(2,5-dimethylphenyl)thiophene (a) obtained as described above (0.67 g, 3.6 mmoles) in anhydrous tetrahydrofuran (THF) (Aldrich) (15 ml), ), at -78°C, by dripping: the obtained reaction mixture was kept under stirring and the temperature was brought to -55°C in 3 hours. Subsequently, after placing the flask in a bath containing acetone (Aldrich) and dry ice at -78°C, tributylstannyl chloride (Aldrich) was added by dripping (1.4 g; 1.17 ml; 4.32 mmoles). After 15 minutes the flask was removed from the bath, the temperature was allowed to rise to 20°C and the reaction mixture was kept, under stirring, at said temperature, for 12 hours. Subsequently, after the addition of a saturated solution of sodium bicarbonate (Aldrich) (20 ml), the reaction mixture was extracted with ethyl ether (Aldrich) (2 × 20 ml): the obtained organic phase was washed with a saturated solution of sodium bicarbonate (Aldrich) (25 ml) and subsequently dried on sodium sulfate (Aldrich). After removing the residual solvent by distillation under reduced pressure, the obtained residue comprising 2-(2,5-dimethylphenyl)-5-tributylstannylthiophene (b):

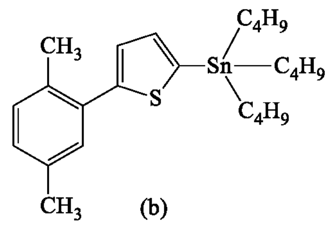

(b)

was used as follows.

In a 50 ml microwave tube, under argon flow, dibromopyridinethiadiazole (Aldrich) (0.42 g; 1.4 mmoles), dimethylformamide (DMF) (Aldrich) (15 ml), the residue comprising 2-(2,5-dimethylphenyl)-5-tributylstannylthiophene (b) obtained as described above and tetrakis(triphenylphosphine)palladium(0) (Aldrich) (0.011 g; $9.5 \times 10^{-3}$ mmoles) are charged: the tube was kept, under argon flow, under stirring, for 30 seconds, at room temperature (25°C). The tube was then sealed and heated, under stirring, in a microwave setting the temperature ramp as follows: from room temperature (25°C) to 120 °C in 2 minutes; 2 minutes at 120°C; from 120°C to 140°C in 2 minutes, 2 minutes at 140°C; from 140°C to 170°C in 2 minutes; 36 minutes at 170°C. Subsequently, the whole was poured into distilled water (40 ml) and extracted with dichloromethane (Aldrich) (3 × 20 ml): the organic phase obtained was washed to neutrality with distilled water (3 × 25 ml) and subsequently dried on sodium sulfate (Aldrich). After having removed most of the residual solvent by distillation under reduced pressure, the obtained residue was added, by dripping, to 50 ml of methanol (Aldrich), obtaining a precipitate which was recovered by filtration and subsequently purified by elution on chromatography column of silica gel [eluent: mixture of *n*-heptane (Aldrich)/dichloromethane (Aldrich) in a ratio of 9/1 (v/v)] obtaining 0.6 g of 4,7-di(5-(1-(2,5-dimethyl)phenyl)thiophen-2-yl)-[1,2,5]thiadiazole[3,4-c]pyridine (PPDTP) (yield = 84%).

EXAMPLE 3

Synthesis of 4,7-di(5-(1-(2,6-dimethyl)phenyl)thiophen-2-yl)-[1,2,5]thiadiazole-[3,4-c]pyridine (MPDTP)

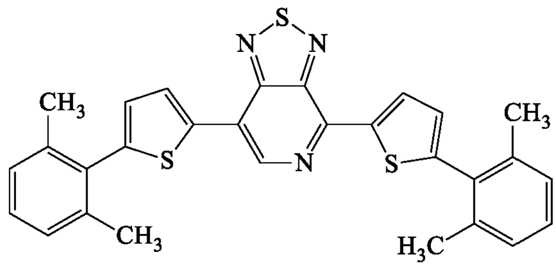

(MPDTP)

(1) Synthesis of 2-(2,6-dimethylphenyl)thiophene (c)

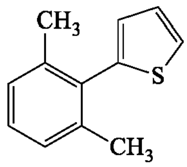

(c)

In a 100 ml flask, equipped with magnetic stirring, thermometer and coolant, in an inert atmosphere, 2,6-dimethylphenylboronic acid (Aldrich) (1.1 g; 7.3 mmoles) and potassium carbonate (Aldrich) (3.4 g; 24.6 mmoles) dissolved in distilled water (10 ml) were added to a solution of 2-bromothiophene (Aldrich) (1 g; 6.13 mmoles) in dioxane (Aldrich) (30 ml). After removing the present air by 3 vacuum/nitrogen cycles, tetrakis(triphenylphosphine)-palladium(0) (Aldrich) (0.14 g; 0.12 mmoles) was added obtaining a reaction mixture which was immersed into a bath pre-heated to 85°C and kept, under stirring, at said temperature, for 16 hours. Subsequently, the obtained reaction mixture, after addition of a saturated aqueous solution of sodium chloride (Aldrich) (50 ml), was extracted with ethyl ether (Aldrich) (3 × 50 ml): the obtained organic phase was washed with neutral with distilled water (3 × 25 ml) and subsequently dried on sodium sulfate (Aldrich). After removing the solvent by distillation under reduced pressure, the obtained residue was purified by elution on a chromatographic column of silica gel [eluent: n-heptane(Aldrich)] obtaining 0.95 g of 2-(2,6-dimethylphenyl)thiophene (c) (yield = 83%).

(2) Synthesis of 4,7-di(5-(1-(2,6-dimethyl)phenyl)thiophen-2-yl)-[1,2,5]thiadiazole[3,4-c]pyridine (MPDTP)

In a 100 ml flask equipped with magnetic stirring, thermometer and coolant, in an inert atmosphere, n-butyllithium (Aldrich) [1.6 M solution in hexane (Aldrich)] (2.5 ml; 4 mmoles) was added to a solution of 2-(2,6-dimethylphenyl)thiophene (c) obtained as described above (0.67 g, 3.6 mmoles) in anhydrous tetrahydrofuran (THF) (Aldrich) (15 ml), at -78°C, by dripping: the obtained reaction mixture was kept under stirring and the temperature was brought to -55°C in 3 hours. Subsequently, after placing the flask in a bath containing acetone (Aldrich) and dry ice at -78°C, tributylstannyl chloride (Aldrich) was added by dripping (1.4 g; 1.17 ml; 4.32 mmoles). After 15 minutes the flask was removed from the bath, the temperature was allowed to rise to 20°C and the reaction mixture was kept, under stirring, at said temperature, for 12 hours. Subsequently, after the addition of a saturated solution of sodium bicarbonate (Aldrich) (20 ml), the reaction mixture was extracted with ethyl ether (Aldrich) (2 × 20 ml): the obtained organic phase was washed with a saturated solution of sodium bicarbonate (Aldrich) (25 ml) and subsequently dried on sodium sulfate (Aldrich). After removing the residual solvent by distillation under reduced pressure, the obtained residue comprising 2-(2,6-dimethylphenyl)-5-tributylstannylthiophene (d):

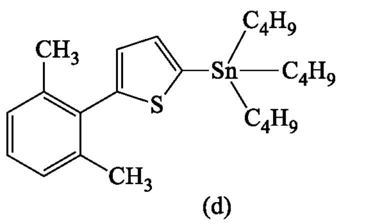

(d)

was used as follows.

In a 50 ml microwave tube, under argon flow, dibromopyridinethiadiazole (Aldrich) (0.42 g; 1.4 mmoles), dimethylformamide (DMF) (Aldrich) (15 ml), the residue comprising 2-(2,6-dimethylphenyl)-5-tributylstannylthiophene (d) obtained as described above and tetrakis (triphenylphosphine)palladium (0) (Aldrich) (0.011 g; $9.5 \times 10^{-3}$ mmoles) are charged: the tube was kept, under argon flow, under stirring, for 30 seconds, at room temperature (25°C). The tube was then sealed and heated, under stirring, in a microwave setting the temperature ramp as follows: from room temperature (25°C) to 120°C in 2 minutes; 2 minutes at 120°C; from 120°C to 140°C in 2 minutes, 2 minutes at 140°C; from 140°C to 170°C in 2 minutes; 36 minutes at 170°C. Subsequently, the whole was poured into distilled water (40 ml) and extracted with dichloromethane (Aldrich) (3 × 20 ml): the organic phase obtained was washed with neutral water (3 × 25 ml) and subsequently dried on sodium sulfate (Aldrich). After having removed most of the residual solvent by distillation under reduced pressure, the obtained residue was added, by dripping, to 50 ml of methanol (Aldrich), obtaining a precipitate which was recovered by filtration and subsequently purified by elution on chromatography column of silica gel [eluent: mixture of n-heptane (Aldrich)/dichloromethane (Aldrich) in a ratio of 9/1 (v/v)] obtaining 0.6 g of 4,7-di(5-(1-(2,6-dimethyl)phenyl)thiophen-2-yl)-[1,2,5]thiadiazole[3,4-c]pyridine (MPDTP) (yield = 95%).

EXAMPLE 4

Synthesis of 4,7-di(5-(1-(2-phenoxy)phenyl)thiophen-2-yl)-[1,2,5]thiadiazole [3,4-c]pyridine (POPDTP)

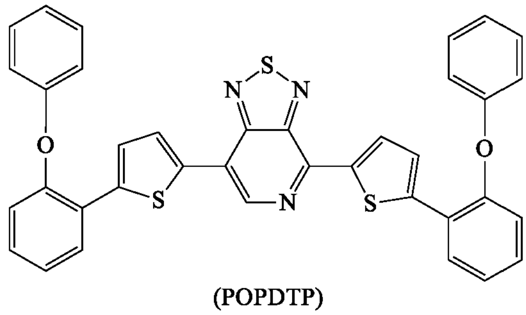

(POPDTP)

(1) Synthesis of 2-(2-phenoxyphenyl)thiophene (e)

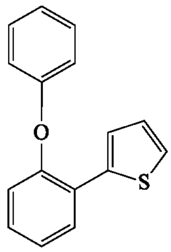

(e)

In a 100 ml flask, equipped with magnetic stirring, thermometer and coolant, in an inert atmosphere, 2-phenoxyphenylboronic acid (Aldrich) (1.6 g; 7.3 mmoles) and potassium carbonate (Aldrich) (3.4 g; 24.6 mmoles) dissolved in distilled water (10 ml) were added to a solution of 2-bromothiophene (Aldrich) (1 g; 6.13 mmoles) in dioxane (Aldrich) (30 ml). After removing the air present by 3 vacuum/nitrogen cycles, tetrakis(triphenylphosphine)-palladium(0) (Aldrich) (0.14 g; 0.12 mmoles) was added obtaining a reaction mixture which was immersed into a bath pre-heated to 85°C and kept, under stirring, at said temperature, for 16 hours. Subsequently, the obtained reaction mixture, after addition of a saturated aqueous solution of sodium chloride (Aldrich) (50 ml), was extracted with ethyl ether (Aldrich) (3 × 25 ml): the obtained organic phase was washed with neutral with distilled water (3 × 25 ml) and subsequently dried on sodium sulfate (Aldrich). After removing the solvent by distillation under reduced pressure, the obtained residue was purified by elution on a chromatographic column of silica gel [eluent: n-heptan (Aldrich)] obtaining 1.4 g of 2-(2-phenoxyphenyl)thiophene (e) (yield = 91%).

(2) Synthesis of 4,7-di(5-(1-(2-phenoxy)phenyl)thiophen-2-yl)-[1,2,5]thiadiazole[3,4-c]pyridine (POPDTP)

In a 100 ml flask equipped with magnetic stirring, thermometer and coolant, in an inert atmosphere, n-butyllithium (Aldrich) [1.6 M solution in hexane (Aldrich)] (1.3 ml; 2.1 mmoles) was added to a solution of 2-(2-phenoxyphenyl)thiophene (e) obtained as described above (0.48 g, 1.9 mmoles) in anhydrous tetrahydrofuran (THF) (Aldrich) (15 ml), at -78°C, by dripping: the reaction mixture obtained was kept under stirring and the temperature was brought to -55°C in 3 hours. Subsequently, after placing the flask in a bath containing acetone (Aldrich) and dry ice at -78°C, tributylstannyl chloride (Aldrich) was added by dripping (0.74 g; 0.62 ml; 2.3 mmoles). After 15 minutes the flask was removed from the bath, the temperature was allowed to rise to 20°C and the reaction mixture was kept, under stirring, at said temperature, for 12 hours. Subsequently, after the addition of a saturated solution of sodium bicarbonate (Aldrich) (20 ml), the reaction mixture was extracted with ethyl ether (Aldrich) (2 × 20 ml): the obtained organic phase was washed with a saturated solution of sodium bicarbonate (Aldrich) (25 ml) and subsequently dried on sodium sulfate (Aldrich). After removing the residual solvent by distillation under reduced pressure, the obtained residue comprising 2-(2-phenoxyphenyl)-5-tributylstannylthiophene (f):

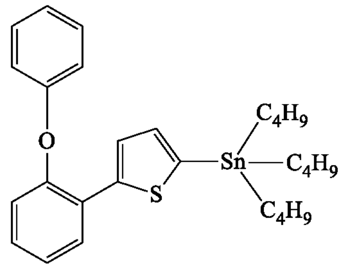

(f)

was used as follows.

In a 50 ml microwave tube, under argon flow, dibromopyridinethiadiazole (Aldrich) (0.225 g; 0.76 mmoles), dimethylformamide (DMF) (Aldrich) (10 ml), the residue comprising 2-(2-phenoxyphenyl)-5-tributylstannylthiophene (f) obtained as described above and tetrakis(triphenylphosphine)palladium(0) (Aldrich) (0,0061 g; $5,3 \times 10^{-3}$ mmoles) are charged: the tube was kept, under argon flow, under stirring, for 30 seconds, at room temperature (25°C). The tube was then sealed and heated, under stirring, in a microwave setting the temperature ramp as follows: from room temperature (25°C) to 120°C in 2 minutes; 2 minutes at 120°C; from 120°C to 140°C in 2 minutes, 2 minutes at 140°C; from 140°C to 170°C in 2 minutes; 36 minutes at 170°C. Subsequently, the whole was poured into distilled water (40 ml) and extracted with dichloromethane (Aldrich) (3 × 20 ml): the organic phase obtained was washed to neutrality with distilled water (3 × 25 ml) and subsequently dried on sodium sulfate (Aldrich). After having removed most of the residual solvent by distillation under reduced pressure, the obtained residue was added, by dripping, to 50 ml of methanol (Aldrich), obtaining a precipitate which was recovered by filtration and subsequently purified by elution on chromatography column of silica gel [eluent: mixture of n-heptane (Aldrich)/dichloromethane (Aldrich) in a ratio of 9/1 (v/v)] obtaining 0.2 g of 4,7-di(5-(1-(2-phenoxy)phenyl)thiophen-2-yl)-[1,2,5]thiadiazole[3,4-c]pyridine (POPDTP) (yield = 46%).

EXAMPLE 5

Synthesis of 4(5-(1-(2-phenoxy)phenyl)thiophen-2-yl)-7-(thiophen-2-yl)Γ1,2,SlthiadiazoleΓ3,4-clpyridine (MonoPOP-DTP)

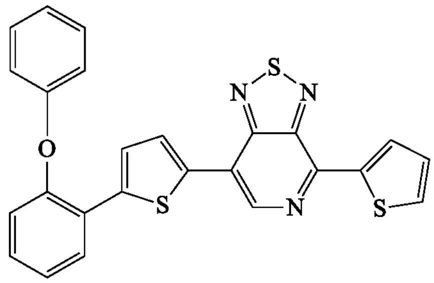

(Mono-POPDTP)

(1) Synthesis of 4(5-(1-(2-bromo)phenyl)thiophen-2-yl)-7-(thiophen-2-yl)[1,2,5]thiadiazole[3,4-c]pyridine (g)

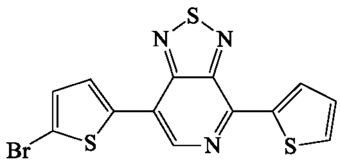

(g)

In a 100 ml flask, equipped with magnetic stirring, thermometer and coolant, in an inert atmosphere, N-bromosuccinimide (Aldrich) (0.694 g; 3.92 mmoles) was added to a solution of 4,7-dithienyl[1,2,5]thiadiazole[3,4-c]pyridine (DTP) obtained as described in Example 1 (0.53 g; 1.76 mmoles) in chloroform (Aldrich) (13.2 ml): the obtained mixture was left, under stirring, at room temperature (25°C), in the dark, for 12 hours. Subsequently, after adding 50 ml of distilled water, a precipitate was obtained which was recovered by filtration, washed with methanol (Aldrich) (200 ml) and recrystallized from a mixture *n*-heptane (Aldrich)/dichloromethane (Aldrich) [ratio 9/1 (v/v)] obtaining 0.549 g of 4(5-(1-(2-bromo)phenyl)thiophen-2-yl)-7-(thiophen-2-yl)[1,2,5]thiadiazole[3,4-c]pyridine (g) (yield = 89%).

(2) Synthesis of 4(5-(1- (2-phenoxy)phenyl)thiophen-2-yl)-7-(thiophen-2-yl)[1,2,5]thiadiazole[3,4-c]pyridine (MonoPOPDTP)

In a 100 ml flask, equipped with magnetic stirring, thermometer and coolant, in an inert atmosphere, 2-phenoxyphenylboronic acid (Aldrich) (0.749 g; 3.5 mmoles) and potassium carbonate (Aldrich) (1.4 g; 10.3 mmoles) dissolved in distilled water (5 ml) were added to a solution of 4(5-(1-(2-bromo)phenyl)thiophen-2-yl)-7-(thiophen-2-yl)[1,2,5]thiadiazole[3,4-c]pyridine (g) obtained as described above (0.549 g; 1.56 mmoles) in dioxane (Aldrich) (30 ml). After removing the air present by 3 vacuum/nitrogen cycles, tetrakis(triphenylphosphine)palladium(0) (Aldrich) (0.072 g; 0.07 mmoles) was added obtaining a reaction mixture which was immersed into a bath pre-heated to 85°C and kept, under stirring, at said temperature, for 16 hours. Subsequently, the obtained reaction mixture, after addition of a saturated aqueous solution of sodium chloride (Aldrich) (30 ml), was extracted with ethyl ether (Aldrich) (3 × 20 ml): the obtained organic phase was washed to neutrality with distilled water (3 × 25 ml) and subsequently dried on sodium sulfate (Aldrich). After removing the solvent by distillation under reduced pressure, the obtained residue was purified by elution on a chromatographic column of silica gel [eluent: n-heptane (Aldrich)] obtaining 0.5 g of 4(5-(1-(2-phenoxy)phenyl)thiophen-2-yl)-7-(thiophen-2-yl)[1,2,5]thiadiazole[3,4-c]pyridine (MonoPOPDTP) (yield = 83%).

EXAMPLE 6

Synthesis of 4,7-di(5-(1-(2,4,6-triphenoxy)phenyl)thiophen-2-yl)-7-(thiophen-2-yl)Γ1,2,SlthiadiazoleΓ3,4-clpyridine (TriPOPDTP)

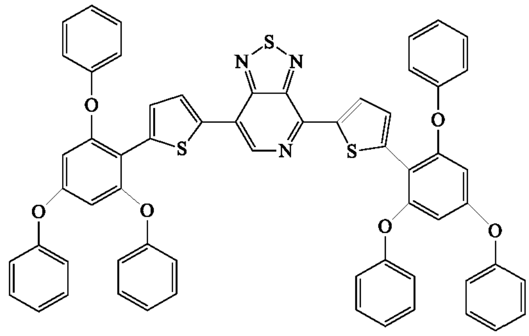

(Tri-POPDTP)

(1) Synthesis of 2,4,6-triphenoxy-1-bromobenzene (h)

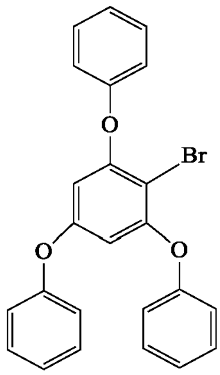

(h)

In a 50 ml microwave tube, under argon flow, 2,4,6-trifluoro-1-bromobenzene (Aldrich) (1.6 g; 0.9 mmoles), phenol (Aldrich) (3.2 ml), potassium carbonate (Aldrich) (5 g; 36.3 mmoles) and N-methyl pyrrolidone (Aldrich) (26 ml) were charged: the tube was kept, under argon flow, under stirring, for 60 seconds, at room temperature (25°C). The tube was then sealed and heated, under stirring, in a microwave setting the temperature ramp as follows: from room temperature (25°C) to 220°C in 4 minutes; 3 hours at 220°C. Subsequently, the whole was poured into distilled water (50 ml) and extracted with ethyl ether (Aldrich) (3 × 25 ml): the obtained organic phase was washed to neutrality with distilled water (3 × 25 ml) and subsequently dried on sodium sulfate (Aldrich). After having removed most of the residual solvent by distillation under reduced pressure, the obtained residue was added, by dripping, to 50 ml of methanol (Aldrich), obtaining a precipitate which was recovered by filtration and subsequently purified by elution on chromatography column of silica gel [eluent: n-heptane (Aldrich)] obtaining 2.3 g of 2,4,6-triphenoxy-1-bromobenzene (h) (yield = 70%).

(2) Synthesis of 2,4,6-triphenoxy-1-(2-thienyl)benzene (i)

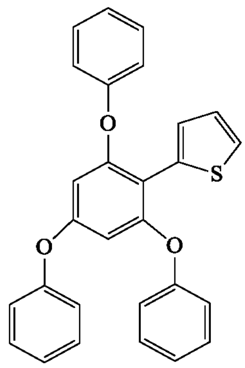

(i)

In a 100 ml flask equipped with magnetic stirring, thermometer and coolant, in an inert atmosphere, 2-tributylstannylthiophene (Aldrich) (1.5 g; 1.3 ml; 4.2 mmoles) was added to a 0.1 M solution of 2,4,6-triphenoxy-1-bromobenzene (h) obtained as described above (1.5 g; 3.5 mmoles) in anhydrous toluene (Aldrich) (13.2 ml). After removing the air present by 3 vacuum/nitrogen cycles, tris-dibenzylideneacetone dipalladium (Aldrich) (0.048 g; 0.05 mmoles) and tris-o-tolylphosphine (Aldrich) were added obtaining a reaction mixture which was immersed into a pre-heated bath at 110°C and kept, under stirring, at said temperature, for 12 hours. Subsequently, the reaction mixture was poured into distilled water (50 ml) and extracted with dichloromethane (Aldrich) (3 × 25 ml): the obtained organic phase was washed to neutrality with distilled water (3 × 25 ml) and subsequently dried on sodium sulfate (Aldrich). After having removed most of the residual solvent by distillation under reduced pressure, the obtained residue was added, by dripping, to 50 ml of methanol (Aldrich), obtaining a precipitate which was recovered by filtration and subsequently purified by elution on chromatography column of silica gel [eluent: n-heptane (Aldrich)] obtaining 1.1 g of 2,4,6-triphenoxy-1-(2-thienyl)benzene (i) (yield = 72%).

(3) Synthesis of 4,7-di(5-(1-(2,4,6-triphenoxy)phenyl)thiophen-2-yl)-7-(thiophen-2-yl)[1,2,Slthiadiazole[3,4-c]pyridine (TriPOPDTP)

In a 100 ml flask, equipped with magnetic stirring, thermometer and coolant, in an inert atmosphere, n-butyllithium (Aldrich) [1.6 M solution in hexane (Aldrich)] (0.8 ml; 1.3 mmoles) was added to a 0.1 M solution of 2,4,6-triphenoxy-1-(2-thienyl)benzene (i) obtained as described above (0.5 g; 1.15 mmoles) in anhydrous tetrahydrofuran (THF) (Aldrich) (11 ml), at -78°C, by dripping: the reaction mixture obtained was kept under stirring and the temperature was brought to -55°C in 3 hours. Subsequently, after placing the flask in a bath containing acetone (Aldrich) and dry ice at -78°C, tributylstannyl chloride (Aldrich) was added by dripping (0.5 g; 0.4 ml; 1.4 mmoles). After 15 minutes the flask was removed from the bath, the temperature was allowed to rise to 20°C and the reaction mixture was kept, under stirring, at said temperature, for 12 hours. Subsequently, after the addition of a saturated solution of sodium bicarbonate (Aldrich) (30 ml), the reaction mixture was extracted with ethyl ether (Aldrich) (2 × 25 ml): the obtained organic phase was washed with a saturated solution of sodium bicarbonate (Aldrich) (25 ml) and subsequently dried on sodium sulfate (Aldrich). After removing the residual solvent by distillation under reduced pressure, the obtained residue comprising 2,4,6-triphenoxy-1-[2'(5'-tributylstannyl)thienyl]benzene (1):

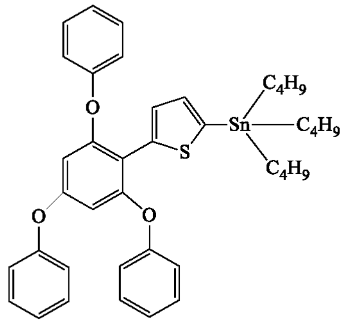

(l)

was used as follows.

In a 50 ml microwave tube, under argon flow, dibromopyridinethiadiazole (Aldrich) (0.15 g; 0.5 mmoles), dimethylformamide (DMF) (Aldrich) (10 ml), the residue comprising 2,4,6-triphenoxy-1-[2'(5'-tributylstannyl)thienyl]benzene (1) obtained as described above and tetrakis(triphenylphosphine)palladium(0) (Aldrich) (0,004 g; $3,4\times10^{-3}$ mmoles) are charged: the tube was kept, under argon flow, under stirring, for 30 seconds, at room temperature (25°C). The tube was then sealed and heated, under stirring, in a microwave setting the temperature ramp as follows: from room temperature (25°C) to 120°C in 2 minutes; 2 minutes at 120°C; from 120°C to 140°C in 2 minutes, 2 minutes at 140°C; from 140°C to 170°C in 2 minutes; 36 minutes at 170°C. Subsequently, the whole was poured into distilled water (30 ml) and extracted with dichloromethane (Aldrich) ($3\times20$ ml): the organic phase obtained was washed to neutrality with distilled water ($3\times25$ ml) and subsequently dried on sodium sulfate (Aldrich). After having removed most of the residual solvent by distillation under reduced pressure, the obtained residue was added, by dripping, to 30 ml of methanol (Aldrich), obtaining a precipitate which was recovered by filtration and subsequently purified by elution on chromatography column of silica gel [eluent: mixture of n-heptane (Aldrich)/dichloromethane (Aldrich) in a ratio of 9/1 (v/v)] obtaining 0.35 g of 4,7-di(5-(1-(2,4,6-triphenoxy)-phenyl)thiophen-2-yl)-7-(thiophen-2-yl)[1,2,5]-thiadiazole-[3,4-c]pyridine (TriPOPDTP) (yield = 70%).

EXAMPLE 7 (comparative)

6 g of polymethylmethacrylate (PMMA) Altuglas VSUVT 100 (Arkema) and 49.5 mg of 4,7-di-(thien-2'-yl)-2,1,3-benzothiadiazole (DTB) obtained as described in the Example 1 of the international patent application WO 2012/007834 in the name of the Applicant, were dissolved in 30 ml of 1,2-dichlorobenzene (Aldrich). The obtained solution was subsequently deposited, evenly, on a polymethylmethacrylate (PMMA) sheet (dimensions 300 mm $\times$ 90 mm $\times$ 6 mm) using a "Doctor Blade" film applicator and the solvent was allowed to evaporate at room temperature (25°C), in a light stream of air, for 24 hours. The result was a transparent sheet of orange colour conferred to it by the film whose thickness was found to be comprised between 100 μm and 50 μm.

An IXYS-KXOB22-12 photovoltaic cell with a surface of 1.2 $cm^2$ was then applied to one of the edges of the polymeric sheet.

The main face of the polymeric sheet [that coated with the thin film containing 4,7-di-(thien-2'-il)-2,1,3-benzothiadiazole (DTB)] was therefore illuminated with a light source of power equal to 1 sun (1000 $W/m^2$) and the electric power generated by the lighting was measured.

The power measurements (P) were made by illuminating a portion of a sheet of dimensions equal to 100 mm $\times$ 90 mm, at a distance (d) increasing from the edge on which the photovoltaic cell was fixed. These measurements at a variable distance from the photovoltaic cell allow the contribution of waveguide, edge, diffusion and self-absorption effects to be quantified.

Figure 1 shows the curve relating to the generated power value (P) expressed in mW (reported on the ordinate), according to the distance (d) from the edge on which the photovoltaic cell was fixed, expressed in cm (reported on the abscissa).

It can be seen how, in the absence of board effects, the average generated power is equal to 5.69 mW (Figure 1).

Figure 2 shows the generated power value (P) expressed in mW (reported on the ordinate) obtained (the number of the example is shown on the abscissa).

EXAMPLE 8 (invention)

6 g of polymethylmethacrylate (PMMA) Altuglas VSUVT 100 (Arkema) and 55.3 mg of 4,7-dithienyl[1,2,5]thiadiazole[3,4-c]pyridine (DTP) obtained as described in Example 1, were dissolved in 30 ml of 1,2-dichlorobenzene (Aldrich). The obtained solution was subsequently deposited, evenly, on a polymethylmethacrylate (PMMA) sheet (dimensions 300 mm $\times$ 90 mm $\times$ 6 mm) using a "Doctor Blade" film applicator and the solvent was allowed to evaporate at room temperature (25°C), in a light stream of air, for 24 hours. The result was a transparent sheet of orange colour conferred to it by the film whose thickness was found to be comprised between 100 μm and 50 μm.

An IXYS-KXOB22-12 photovoltaic cell with a surface of 1.2 $cm^2$ was then applied to one of the edges of the polymeric sheet.

The main face of the polymeric sheet was then illuminated with a light source of power equal to 1 sun (1000 $W/m^2$) [that coated with the thin film containing 4,7-dithienyl[1,2,5]thiadiazole[3,4-c]pyridine (DTP)] and the electrical power generated due to lighting was measured.

The power measurements (P) were made by illuminating a portion of a sheet of dimensions equal to 100 mm $\times$ 90 mm, at a distance (d) increasing from the edge on which the photovoltaic cell was fixed. These measurements at a variable distance from the photovoltaic cell allow the contribution of waveguide, edge, diffusion and self-absorption effects to be quantified.

Figure 1 shows the curve relating to the generated power value (P) expressed in mW (reported on the ordinate),

according to the distance (d) from the edge on which the photovoltaic cell was fixed, expressed in cm (reported on the abscissa).

It can be seen how, in the absence of board effects, the average generated power is equal to 6.47 mW (Figure 1).

Figure 2 shows the generated power value (P) expressed in mW (reported on the ordinate) obtained (the number of the example is shown on the abscissa).

EXAMPLE 9 (invention)

6 g of polymethylmethacrylate (PMMA) Altuglas VSUVT 100 (Arkema) and 89.6 mg of 4,7-di(5-(1-(2,6-dimethyl)phenyl)thiophen-2-yl)-[1,2,5]thiadiazole[3,4-c]pyridine (MPDTP) obtained as described in Example 3, were dissolved in 30 ml of 1,2-dichlorobenzene (Aldrich). The obtained solution was subsequently deposited, evenly, on a polymethylmethacrylate (PMMA) sheet (dimensions 300 mm × 90 mm × 6 mm) using a "Doctor Blade" film applicator and the solvent was allowed to evaporate at room temperature (25°C), in a light stream of air, for 24 hours. The result was a transparent sheet of orange colour conferred to it by the film whose thickness was found to be comprised between 100 μm and 50 μm.

An IXYS-KXOB22-12 photovoltaic cell with a surface of 1.2 $cm^2$ was then applied to one of the edges of the polymeric sheet.

The main face of the polymeric sheet was illuminated with a light source of power equal to 1 sun (1000 $W/m^2$) [that coated with the thin film containing 4,7-di(5-(1-(2,6-dimethyl)phenyl)thiophen-2-yl)-[1,2,5]thiadiazole [3,4-c]-pyridine (MPDTP)] and the electric power generated due to lighting was measured.

The power measurements (P) were made by illuminating a portion of a sheet of dimensions equal to 100 mm × 90 mm, at a distance (d) increasing from the edge on which the photovoltaic cell was fixed. These measurements at a variable distance from the photovoltaic cell allow the contribution of waveguide, edge, diffusion and self-absorption effects to be quantified.

Figure 1 shows the curve relating to the generated power value (P) expressed in mW (reported on the ordinate), according to the distance (d) from the edge on which the photovoltaic cell was fixed, expressed in cm (reported on the abscissa).

It can be seen how, in the absence of board effects, the average generated power is equal to 11.81 mW (Figure 1).

Figure 2 shows the generated power value (P) expressed in mW (reported on the ordinate) obtained (the number of the example is shown on the abscissa).

EXAMPLE 10 (invention)

6 g of polymethylmethacrylate (PMMA) Altuglas VSUVT 100 (Arkema) and 45 mg of 4,7-di(5-(1-(2,5-dimethyl)phenyl)thiophen-2-yl)-[1,2,5 ]thiadiazole [3,4-c]pyridine (PPDTP) obtained as described in Example 2, were dissolved in 30 ml of 1,2-dichlorobenzene (Aldrich). The obtained solution was subsequently deposited, evenly, on a polymethylmethacrylate (PMMA) sheet (dimensions 300 mm × 90 mm × 6 mm) using a "Doctor Blade" film applicator and the solvent was allowed to evaporate at room temperature (25°C), in a light air draft, for 24 hours. The result was a transparent sheet of orange colour conferred to it by the film whose thickness was found to be comprised between 100 μm and 50 μm.

An IXYS-KXOB22-12 photovoltaic cell with a surface of 1.2 $cm^2$ was then applied to one of the edges of the polymeric sheet.

The main face of the polymeric sheet was illuminated with a light source of power equal to 1 sun (1000 $W/m^2$) [that coated with the thin film containing 4,7-di(5-(1-(2,5-dimethyl)phenyl)thiophen-2-yl)-[1,2,5]thiadiazole[3,4-c]-pyridine (PPDTP)] and the electric power generated due to lighting was measured.

The power measurements (P) were made by illuminating a portion of a sheet of dimensions equal to 100 mm × 90 mm, at a distance (d) increasing from the edge on which the photovoltaic cell was fixed. These measurements at a variable distance from the photovoltaic cell allow the contribution of waveguide, edge, diffusion and self-absorption effects to be quantified.

Figure 1 shows the curve relating to the generated power value (P) expressed in mW (reported on the ordinate), according to the distance (d) from the edge on which the photovoltaic cell was fixed, expressed in cm (reported on the abscissa).

It can be seen how, in the absence of board effects, the average generated power is equal to 8.85 mW (Figure 1).

Figure 2 shows the generated power value (P) expressed in mW (reported on the ordinate) obtained (the number of the example is shown on the abscissa).

EXAMPLE 11 (invention)

6 g of polymethylmethacrylate (PMMA) Altuglas VSUVT 100 (Arkema) and 85.4 mg of 4,7-di(5-(1-(2-phenoxy)phenyl)thiophen-2-yl)-[1,2,5]-thiadiazole[3,4-c]pyridine (POPDTP) obtained as described in Example 4, were dissolved in 30 ml of 1,2-dichlorobenzene (Aldrich). The obtained solution was subsequently deposited, evenly, on a polymethylmethacrylate (PMMA) sheet (dimensions 300 mm × 90 mm × 6 mm) using a "Doctor Blade" film applicator and the solvent was allowed to evaporate at room temperature (25°C), in a light stream of air, for 24 hours. The result was a transparent sheet of orange colour conferred to it by the film whose thickness was found to be comprised between 100 μm and 50 μm.

An IXYS-KXOB22-12 photovoltaic cell with a surface of 1.2 $cm^2$ was then applied to one of the edges of the polymeric sheet.

The main face of the polymeric sheet was illuminated with a light source of power equal to 1 sun (1000 $W/m^2$) [that coated with the thin film containing 4,7-di(5-(1-(2-phenoxy)phenyl)thiophen-2-yl)-[1,2,5]thiadiazole[3,4-c]pyridine (POPDTP)] and the electric power generated by lighting was measured.

The power measurements (P) were made by illuminating a portion of a sheet of dimensions equal to 100 mm × 90 mm, at a distance (d) increasing from the edge on which the photovoltaic cell was fixed. These measurements at a variable distance from the photovoltaic cell allow the contribution of waveguide, edge, diffusion and self-absorption effects to be quantified.

Figure 1 shows the curve relating to the generated power value (P) expressed in mW (reported on the ordinate), according to the distance (d) from the edge on which the photovoltaic cell was fixed, expressed in cm (reported on the abscissa).

It can be seen how, in the absence of board effects, the average generated power is equal to 7.57 mW (Figure 1).

Figure 2 shows the generated power value (P) expressed in mW (reported on the ordinate) obtained (the number of the example is shown on the abscissa).

EXAMPLE 12 (invention)

6 g of polymethylmethacrylate (PMMA) Altuglas VSUVT 100 (Arkema) and 60.1 mg of 4,7-di(5-(1-(2-phenoxy)phenyl)thiophen-2-yl)[1,2,5]-thiadiazole[3,4-c]pyridine (POPDTP) obtained as described in Example 4, were dissolved in 30 ml of 1,2-dichlorobenzene (Aldrich). The obtained solution was subsequently deposited, evenly, on a polymethylmethacrylate (PMMA) sheet (dimensions 300 mm × 90 mm × 6 mm) using a "Doctor Blade" film applicator and the solvent was allowed to evaporate at room temperature (25°C), in a light stream of air, for 24 hours. The result was a transparent sheet of orange colour conferred to it by the film whose thickness was found to be comprised between 100 μm and 50 μm.

An IXYS-KXOB22-12 photovoltaic cell with a surface of 1.2 $cm^2$ was then applied to one of the edges of the polymeric sheet.

The main face of the polymeric sheet was illuminated with a light source of power equal to 1 sun (1000 W/m2) [that coated with the thin film containing 4,7-di(5-(1-(2-phenoxy)phenyl)thiophen-2-yl)[1,2,5]thiadiazole[3,4-c]pyridine (POPDTP)] and the electric power generated by lighting was measured.

The power measurements (P) were made by illuminating a portion of a sheet of dimensions equal to 100 mm × 90 mm, at a distance (d) increasing from the edge on which the photovoltaic cell was fixed. These measurements at a variable distance from the photovoltaic cell allow the contribution of waveguide, edge, diffusion and self-absorption effects to be quantified.

Figure 1 shows the curve relating to the generated power value (P) expressed in mW (reported on the ordinate), according to the distance (d) from the edge on which the photovoltaic cell was fixed, expressed in cm (reported on the abscissa).

It can be seen how, in the absence of board effects, the average generated power is equal to 8.06 mW (Figure 1).

Figure 2 shows the generated power value (P) expressed in mW (reported on the ordinate) obtained (the number of the example is shown on the abscissa).

EXAMPLE 13 (invention)

6 g of polymethylmethacrylate (PMMA) Altuglas VSUVT 100 (Arkema) and 168.3 mg of 4,7-di(5-(1-(2,4,6-triphenoxy)phenyl)thiophen-2-yl)-7-(thiophen-2-yl)[1,2,5]thiadiazole[3,4-c]pyridine (TriPOPDTP) obtained as described in Example 6, were dissolved in 30 ml of 1,2-dichlorobenzene (Aldrich). The obtained solution was subsequently deposited, evenly, on a polymethylmethacrylate (PMMA) sheet (dimensions 300 mm × 90 mm × 6 mm) using a "Doctor Blade" film applicator and the solvent was allowed to evaporate at room temperature (25°C), in a light stream of air, for 24 hours. The result was a transparent sheet of orange colour conferred to it by the film whose thickness was found to

be comprised between 100 μm and 50 μm.

An IXYS-KXOB22-12 photovoltaic cell with a surface of 1.2 $cm^2$ was then applied to one of the edges of the polymeric sheet.

The main face of the polymeric sheet was illuminated with a light source of power equal to 1 sun (1000 $W/m^2$) [that coated with the thin film containing 4,7-di(5-(1-(2,4,6-triphenoxy)phenyl)thiophen-2-yl)-7-(thiophen-2-yl)[1,2,5]-thiadiazole[3,4-c]pyridine (TriPOPDTP)] and the electric power generated by lighting was measured.

The power measurements (P) were made by illuminating a portion of a sheet of dimensions equal to 100 mm × 90 mm, at a distance (d) increasing from the edge on which the photovoltaic cell was fixed. These measurements at a variable distance from the photovoltaic cell allow the contribution of waveguide, edge, diffusion and self-absorption effects to be quantified.

Figure 1 shows the curve relating to the generated power value (P) expressed in mW (reported on the ordinate), according to the distance (d) from the edge on which the photovoltaic cell was fixed, expressed in cm (reported on the abscissa).

It can be seen how, in the absence of board effects, the average generated power is equal to 6.08 mW (Figure 1).

Figure 2 shows the generated power value (P) expressed in mW (reported on the ordinate) obtained (the number of the example is shown on the abscissa).

## Claims

1. Luminescent solar concentrator (LSC) comprising at least one dithienylpyridinethiadiazole compound having general formula (I) or (II):

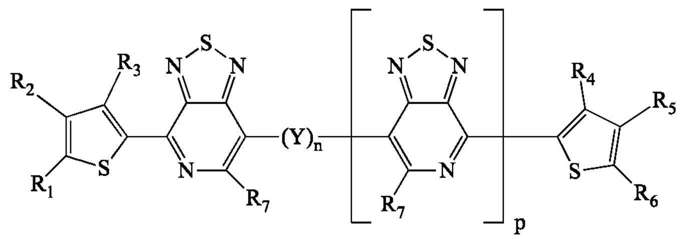

(I)

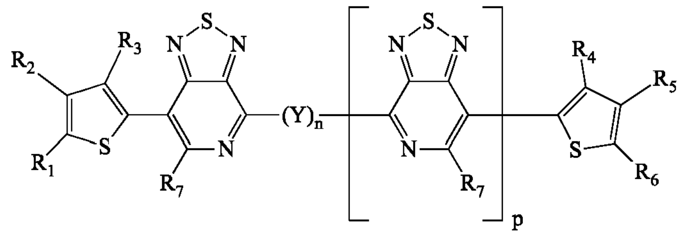

(II)

wherein:

- $R_1$, $R_2$, $R_3$, $R_4$, $R_5$ and $R_6$, mutually identical or different, represent a hydrogen atom; or they are selected from linear or branched, saturated or unsaturated $C_1$-$C_{20}$, preferably $C_1$-$C_8$, alkyl groups, optionally containing heteroatoms, optionally substituted aryl groups, optionally substituted heteroaryl groups;
- $R_7$, mutually identical or different, represent a hydrogen atom; or they are selected from linear or branched, saturated or unsaturated, $C_1$-$C_{20}$, preferably $C_1$-$C_8$, alkyl groups, optionally containing heteroatoms, optionally substituted aryl groups, optionally substituted heteroaryl groups, optionally substituted phenoxy groups;
- or $R_1$ and $R_2$ or $R_2$ and $R_3$ and/or $R_4$ and $R_5$ or $R_5$ and $R_6$, can be optionally linked together so as to form, together with the carbon atoms to which they are linked, a saturated, unsaturated, or aromatic, cycle or polycyclic system containing from 3 to 14 carbon atoms, preferably from 4 to 6 carbon atoms, optionally containing one or more heteroatoms such as oxygen, sulfur, nitrogen, silicon, phosphorus, selenium;
- Y represents a divalent cycle or polycyclic group containing one or more aromatic or heteroaromatic rings, said aromatic or heteroaromatic rings being optionally substituted;
- n is 0 or 1;
- p is 0 or 1.

**2.** Luminescent solar concentrator (LSC) according to claim 1, wherein in said general formula (I):

- $R_1$, $R_2$, $R_3$, $R_4$, $R_5$, $R_6$ and $R_7$, mutually identical, represent a hydrogen atom; n and p are 0; or
- $R_2$, $R_3$, $R_4$, $R_5$ and $R_7$, mutually identical, represent a hydrogen atom; $R_1$ and $R_6$, mutually identical, are selected from optionally substituted aryl groups, preferably they are a 2,5-dimethylphenyl group; n and p are 0; or
- $R_2$, $R_3$, $R_4$, $R_5$ and $R_7$, mutually identical, represent a hydrogen atom; $R_1$ and $R_6$, mutually identical, are selected from optionally substituted aryl groups, preferably they are a 2,6-dimethylphenyl group; n and p are 0; or
- $R_2$, $R_3$, $R_4$, $R_5$ and $R_7$, mutually identical, represent a hydrogen atom; $R_1$ and $R_6$, mutually identical, are selected from optionally substituted aryl groups, preferably they are a 2-phenoxyphenyl group; n and p are 0; or
- $R_2$, $R_3$, $R_4$, $R_5$, $R_6$ and $R_7$, mutually identical, represent a hydrogen atom; $R_1$ is selected from optionally substituted aryl groups, preferably it is a 2-phenoxyphenyl group; n and p are 0; or
- $R_2$, $R_3$, $R_4$, $R_5$ and $R_7$, mutually identical, represent a hydrogen atom; $R_1$ and $R_6$, are selected from optionally substituted aryl groups, preferably they are a 2,4,6-phenoxyphenyl group; n and p are 0.

**3.** Use of at least one dithienylpyridinethiadiazole compound having general formula (I) or (II) according to claim 1 or 2, in the construction of luminescent solar concentrators (LSCs).

**4.** Photovoltaic device (or solar device) comprising at least one photovoltaic cell (or solar cell), and at least one luminescent solar concentrator (LSC) comprising at least one dithienylpyridinethiadiazole compound having general formula (I) or (II) according to claim 1 or 2.

**5.** Dithienylpyridinethiadiazole compound having general formula (I) or (II):

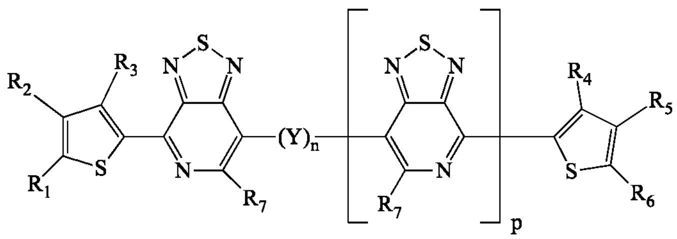

(I)

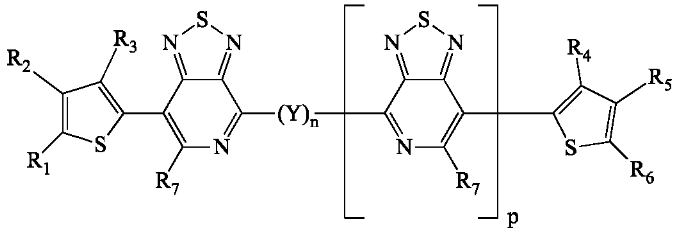

(II)

wherein:

- $R_2$, $R_3$, $R_4$, $R_5$ and $R_7$, mutually identical, represent a hydrogen atom; $R_1$ and $R_6$, mutually identical, are a 2,5-dimethylphenyl group; n and p are 0; or
- $R_2$, $R_3$, $R_4$, $R_5$ and $R_7$, mutually identical, represent a hydrogen atom; $R_1$ and $R_6$, mutually identical, are a 2,6-dimethylphenyl group; n and p are 0; or
- $R_2$, $R_3$, $R_4$, $R_5$ and $R_7$, mutually identical, represent a hydrogen atom; $R_1$ and $R_6$, mutually identical, are a 2-phenoxyphenyl group; n and p are 0; or
- $R_2$, $R_3$, $R_4$, $R_5$, $R_6$ and $R_7$, mutually identical, represent a hydrogen atom; $R_1$ is a 2-phenoxyphenyl group; n and p are 0; or
- $R_2$, $R_3$, $R_4$, $R_5$ and $R_7$, mutually identical, represent a hydrogen atom; $R_1$ and $R_6$, are a 2,4,6-phenoxyphenyl group; n and p are 0.

**Patentansprüche**

**1.** Lumineszierender Solarkonzentrator (LSC), umfassend mindestens eine Dithienylpyridinthiadiazol-Verbindung mit

der allgemeinen Formel (I) oder (II):

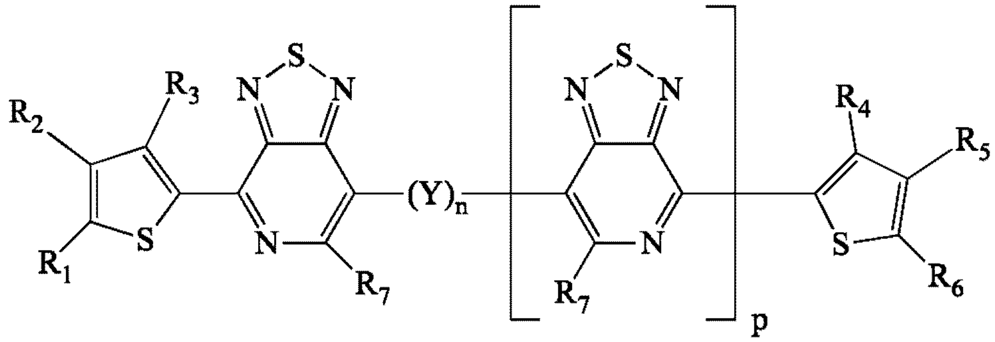

(I)

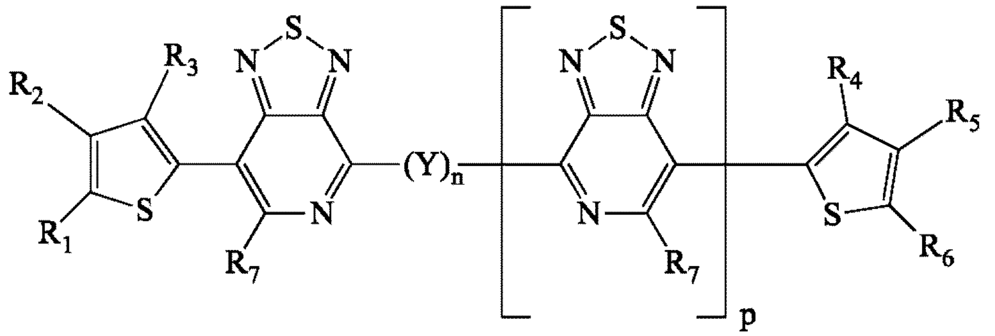

(II)

wobei:

$R_1$, $R_2$, $R_3$, $R_4$, $R_5$ und $R_6$, die untereinander identisch oder verschieden sind, ein Wasserstoffatom darstellen; oder ausgewählt sind aus linearen oder verzweigten, gesättigten oder ungesättigten $C_1$-$C_{20}$-, vorzugsweise $C_1$-$C_8$-, Alkylgruppen, die optional Heteroatome enthalten, optional substituierten Arylgruppen, optional substituierten Heteroarylgruppen;

$R_7$, die untereinander identisch oder verschieden sind, ein Wasserstoffatom darstellen; oder ausgewählt sind aus linearen oder verzweigten, gesättigten oder ungesättigten $C_1$-$C_{20}$-, vorzugsweise $C_1$-$C_8$-, Alkylgruppen, die optional Heteroatome enthalten, optional substituierten Arylgruppen, optional substituierten Heteroarylgruppen, optional substituierten Phenoxygruppen;

oder $R_1$ und $R_2$ oder $R_2$ und $R_3$ und/oder $R_4$ und $R_5$ oder $R_5$ und $R_6$ optional miteinander verbunden sein können, um zusammen mit den Kohlenstoffatomen, an die sie gebunden sind, ein gesättigtes, ungesättigtes oder aromatisches, zyklisches oder polyzyklisches System, enthaltend von 3 bis 14 Kohlenstoffatome, vorzugsweise von 4 bis 6 Kohlenstoffatome, zu bilden, das optional ein oder mehrere Heteroatome, wie Sauerstoff, Schwefel, Stickstoff, Silizium, Phosphor, Selen, enthält;

Y eine zweiwertige zyklische oder polyzyklische Gruppe darstellt, die einen oder mehrere aromatische oder heteroaromatische Ringe enthält, wobei die aromatischen oder heteroaromatischen Ringe optional substituiert sind;

n 0 oder 1 ist;

p 0 oder 1 ist.

**2.** Lumineszierender Solarkonzentrator (LSC) nach Anspruch 1, wobei in der allgemeinen Formel (I):

$R_1$, $R_2$, $R_3$, $R_4$, $R_5$, $R_6$ und $R_7$, die untereinander identisch sind, ein Wasserstoffatom darstellen; n und p 0 sind; oder

$R_2$, $R_3$, $R_4$, $R_5$ und $R_7$, die untereinander identisch sind, ein Wasserstoffatom darstellen; $R_1$ und $R_6$, die untereinander identisch sind, aus optional substituierten Arylgruppen ausgewählt sind, vorzugsweise eine 2,5-Dimethylphenylgruppe sind; n und p 0 sind; oder

$R_2$, $R_3$, $R_4$, $R_5$ und $R_7$, die untereinander identisch sind, ein Wasserstoffatom darstellen; $R_1$ und $R_6$, die untereinander identisch sind, aus optional substituierten Arylgruppen ausgewählt sind, vorzugsweise eine 2,6-Dimethylphenylgruppe sind; n und p 0 sind; oder

$R_2$, $R_3$, $R_4$, $R_5$ und $R_7$, die untereinander identisch sind, ein Wasserstoffatom darstellen; $R_1$ und $R_6$, die unter-

einander identisch sind, aus optional substituierten Arylgruppen ausgewählt sind, vorzugsweise eine 2-Phenoxyphenylgruppe sind; n und p 0 sind; oder
$R_2$, $R_3$, $R_4$, $R_5$, $R_6$ und $R_7$, die untereinander identisch sind, ein Wasserstoffatom darstellen; $R_1$ aus optional substituierten Arylgruppen ausgewählt ist, vorzugsweise eine 2-Phenoxyphenylgruppe ist; n und p 0 sind; oder
$R_2$, $R_3$, $R_4$, $R_5$ und $R_7$, die untereinander identisch sind, ein Wasserstoffatom darstellen; $R_1$ und $R_6$ aus optional substituierten Arylgruppen ausgewählt sind, vorzugsweise eine 2,4,6-Phenoxyphenylgruppe sind; n und p 0 sind.

3. Verwendung von mindestens einer Dithienylpyridinthiadiazol-Verbindung mit der allgemeinen Formel (I) oder (II) nach Anspruch 1 oder 2 bei der Herstellung von lumineszierenden Solarkonzentratoren (LSCs).

4. Photovoltaikvorrichtung (oder Solarvorrichtung), umfassend mindestens eine Photovoltaikzelle (oder Solarzelle) und mindestens einen lumineszierenden Solarkonzentrator (LSC), der mindestens eine Dithienylpyridinthiadiazol-Verbindung mit der allgemeinen Formel (I) oder (II) nach Anspruch 1 oder 2 umfasst.

5. Dithienylpyridinthiadiazol-Verbindung mit der allgemeinen Formel (I) oder (II):

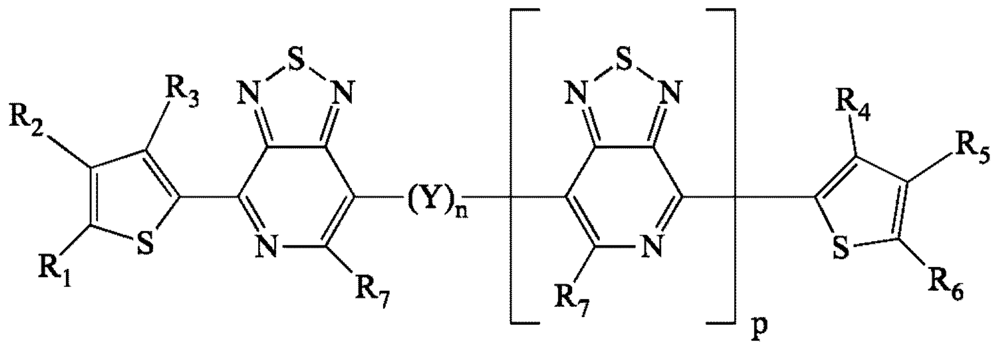

(I)

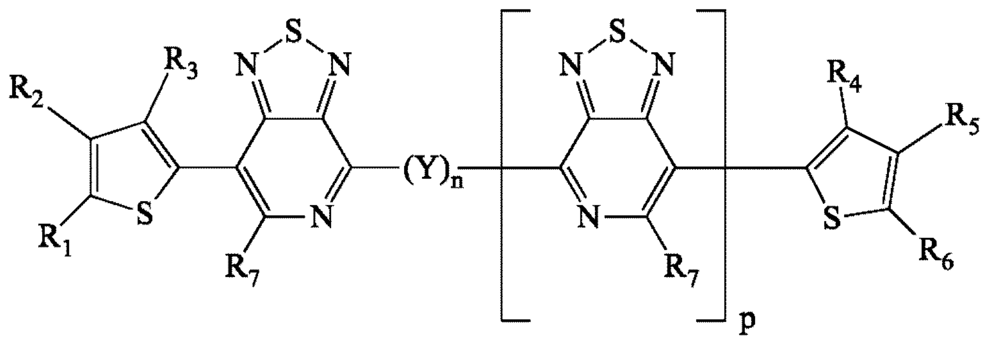

(II)

wobei:

$R_2$, $R_3$, $R_4$, $R_5$ und $R_7$, die untereinander identisch sind, ein Wasserstoffatom darstellen; $R_1$ und $R_6$, die untereinander identisch sind, eine 2,5-Dimethylphenylgruppe sind; n und p 0 sind; oder
$R_2$, $R_3$, $R_4$, $R_5$ und $R_7$, die untereinander identisch sind, ein Wasserstoffatom darstellen; $R_1$ und $R_6$, die untereinander identisch sind, eine 2,6-Dimethylphenylgruppe sind; n und p 0 sind; oder
$R_2$, $R_3$, $R_4$, $R_5$ und $R_7$, die untereinander identisch sind, ein Wasserstoffatom darstellen; $R_1$ und $R_6$, die untereinander identisch sind, eine 2-Phenoxyphenylgruppe sind; n und p 0 sind; oder
$R_2$, $R_3$, $R_4$, $R_5$, $R_6$ und $R_7$, die untereinander identisch sind, ein Wasserstoffatom darstellen; $R_1$ eine 2-Phenoxyphenylgruppe ist; n und p 0 sind; oder
$R_2$, $R_3$, $R_4$, $R_5$ und $R_7$, die untereinander identisch sind, ein Wasserstoffatom darstellen; $R_1$ und $R_6$ eine 2,4,6-Phenoxyphenylgruppe sind; n und p 0 sind.

## Revendications

1. Concentrateur solaire luminescent (LSC) comprenant au moins un composé dithiénylpyridinethiadiazole présentant une formule générale (I) ou (II) :

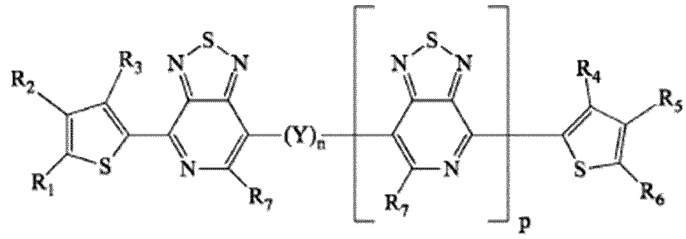

(I)

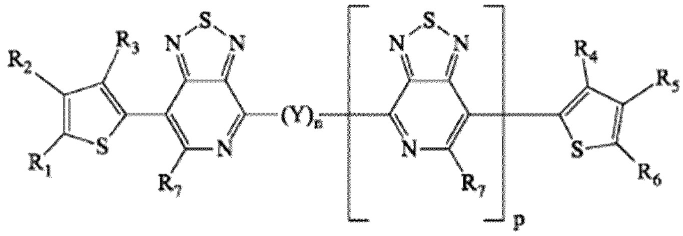

(II)

dans lequel :

- $R_1$, $R_2$, $R_3$, $R_4$, $R_5$ et $R_6$, mutuellement identiques ou différents, représentent un atome d'hydrogène ; ou ils sont sélectionnés parmi des groupes alkyle en $C_1$-$C_{20}$, de préférence $C_1$-$C_8$, linéaires ou ramifiés, saturés ou insaturés, contenant éventuellement des hétéroatomes, des groupes aryle éventuellement substitués, des groupes hétéroaryle éventuellement substitués ;
- $R_7$, mutuellement identiques ou différents, représentent un atome d'hydrogène ; ou ils sont sélectionnés parmi des groupes alkyle en $C_1$-$C_{20}$, de préférence $C_1$-$C_8$, linéaires ou ramifiés, saturés ou insaturés, contenant éventuellement des hétéroatomes, des groupes aryle éventuellement substitués, des groupes hétéroaryle éventuellement substitués, des groupes phénoxy éventuellement substitués ;
- ou $R_1$ et $R_2$ ou $R_2$ et $R_3$ et/ou $R_4$ et $R_5$ ou $R_5$ et $R_6$, peuvent être éventuellement liés les uns aux autres de manière à former, conjointement avec les atomes de carbone auxquels ils sont liés, un cycle ou un système polycyclique saturé, insaturé, ou aromatique contenant de 3 à 14 atomes de carbone, de préférence de 4 à 6 atomes de carbone, contenant éventuellement un ou plusieurs hétéroatomes tels que l'oxygène, le soufre, l'azote, le silicium, phosphore, le sélénium ;
- Y représente un cycle divalent ou un groupe polycyclique contenant un ou plusieurs noyaux aromatiques ou hétéroaromatiques, lesdits noyaux aromatiques ou hétéroaromatiques étant éventuellement substitués ;
- n est 0 ou 1 ;
- p est 0 ou 1.

**2.** Concentrateur solaire luminescent (LSC) selon la revendication 1, dans lequel dans ladite formule générale (I) :

- $R_1$, $R_2$, $R_3$, $R_4$, $R_5$, $R_6$ et $R_7$, mutuellement identiques, représentent un atome d'hydrogène ; n et p sont 0 ; ou
- $R_2$, $R_3$, $R_4$, $R_5$ et $R_7$, mutuellement identiques, représentent un atome d'hydrogène ; $R_1$ et $R_6$, mutuellement identiques, sont sélectionnés parmi des groupes aryle éventuellement substitués, de préférence ils sont un groupe 2,5-diméthylphényle ; n et p sont 0 ; ou
- $R_2$, $R_3$, $R_4$, $R_5$ et $R_7$, mutuellement identiques, représentent un atome d'hydrogène ; $R_1$ et $R_6$, mutuellement identiques, sont sélectionnés parmi des groupes aryle éventuellement substitués, de préférence ils sont un groupe 2,6-diméthylphényle ; n et p sont 0 ; ou
- $R_2$, $R_3$, $R_4$, $R_5$ et $R_7$, mutuellement identiques, représentent un atome d'hydrogène ; $R_1$ et $R_6$, mutuellement identiques, sont sélectionnés parmi des groupes aryle éventuellement substitués, de préférence ils sont un groupe 2-phénoxyphényle ; n et p sont 0 ; ou
- $R_2$, $R_3$, $R_4$, $R_5$ et $R_7$, mutuellement identiques, représentent un atome d'hydrogène ; $R_1$ est sélectionné parmi des groupes aryle éventuellement substitués, de préférence il est un groupe 2-phénoxyphényle ; n et p sont 0 ; ou
- $R_2$, $R_3$, $R_4$, $R_5$ et $R_7$, mutuellement identiques, représentent un atome d'hydrogène ; $R_1$ et $R_6$ sont sélectionnés parmi des groupes aryle éventuellement substitués, de préférence ils sont un groupe 2,4,6-phénoxyphényle ; n et p sont 0.

**3.** Utilisation d'au moins un composé dithiénylpyridinethiadiazole présentant la formule générale (I) ou (II) selon la revendication 1 ou 2, dans la construction de concentrateurs solaires luminescents (LSC).

**4.** Dispositif photovoltaïque (ou dispositif solaire) comprenant au moins une cellule photovoltaïque (ou une cellule solaire), et au moins un concentrateur solaire luminescent (LSC) comprenant au moins un composé dithiénylpyridinethiadiazole présentant la formule générale (I) ou (II) selon la revendication 1 ou 2.

**5.** Composé dithiénylpyridinethiadiazole présentant la formule générale (I) ou (II) :

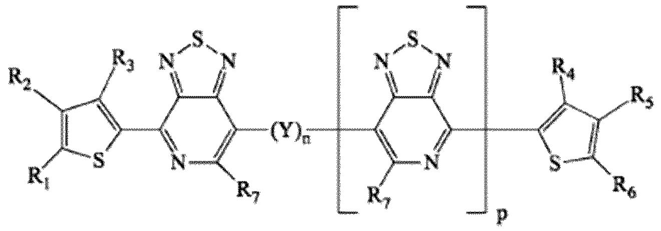

(I)

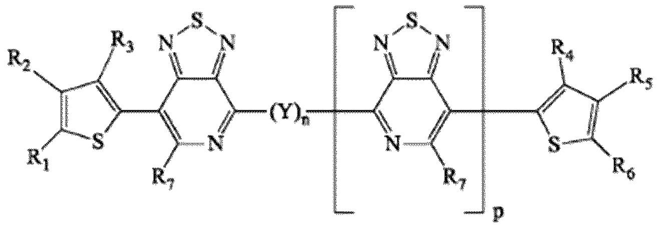

(II)

dans lequel :

- $R_2$, $R_3$, $R_4$, $R_5$ et $R_7$, mutuellement identiques, représentent un atome d'hydrogène ; $R_1$ et $R_6$, mutuellement identiques, sont un groupe 2,5-diméthylphényle ; n et p sont 0 ; ou
- $R_2$, $R_3$, $R_4$, $R_5$ et $R_7$, mutuellement identiques, représentent un atome d'hydrogène ; $R_1$ et $R_6$, mutuellement identiques, sont un groupe 2,6-diméthylphényle ; n et p sont 0 ; ou
- $R_2$, $R_3$, $R_4$, $R_5$ et $R_7$, mutuellement identiques, représentent un atome d'hydrogène ; $R_1$ et $R_6$, mutuellement identiques, sont un groupe 2-phénoxyphényle ; n et p sont 0 ; ou
- $R_2$, $R_3$, $R_4$, $R_5$ et $R_7$, mutuellement identiques, représentent un atome d'hydrogène ; $R_1$ est un groupe 2-phénoxyphényle ; n et p sont 0 ; ou
- $R_2$, $R_3$, $R_4$, $R_5$ et $R_7$, mutuellement identiques, représentent un atome d'hydrogène ; $R_1$ et $R_6$ sont un groupe 2,4,6-phénoxyphényle ; n et p sont 0.

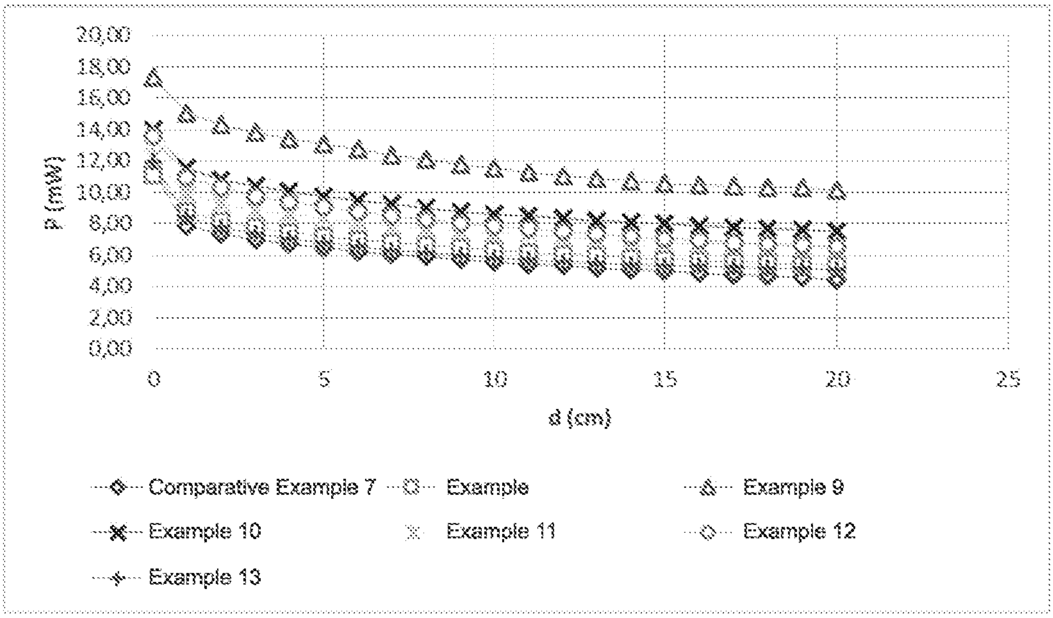
P (mW)
20,00
18,00
16,00
14,00
12,00
10,00
8,00
6,00
4,00
2,00
0,00
0
5
10
15
20
25
d (cm)
Comparative Example 7
Example
Example 9
Example 10
Example 11
Example 12
Example 13

FIG. 1

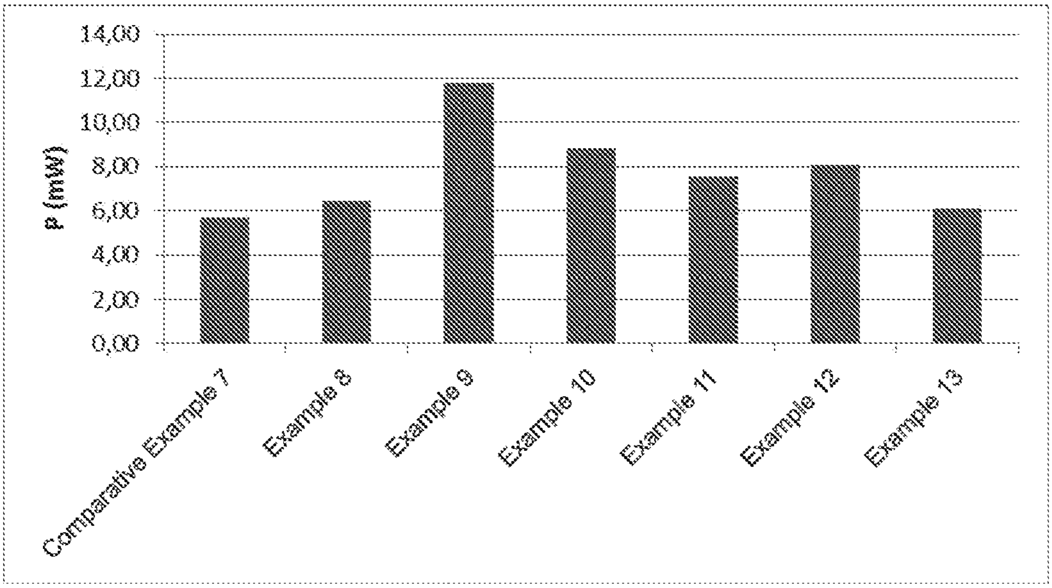
P (mW)
14,00
12,00
10,00
8,00
6,00
4,00
2,00
0,00
Comparative Example 7
Example 8
Example 9
Example 10
Example 11
Example 12
Example 13

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- IT 102018000020419
- WO 2011048458 A
- US 20140167002 A
- US 2011028656 A
- WO 2017118464 A
- IT MI2018000000667
- WO 2012007834 A


### Non-patent literature cited in the description


- **BATHULA C.** *Journal of Fluorescence,* 2016, vol. 26 (3), 1045-1052
- **WELCH G. C.** *Journal of Materials Chemistry,* 2011, vol. 21, 12700-12709
- **BAZAN G. C.** *Journal of the American Chemical Society,* 2013, vol. 135 (6), 2298-2305
- Effects of alkyl side chain and electron-withdrawing group on benzo[1,2,5]thiadiazole-thiophene-based small molecules in organic photovoltaic cells. **ARA C. et al.** Journal Of Materials Science. Kluwer Academic Publishers, 18 April 2016, vol. 51, 6770-6780
- **MATEKER W. et al.** Molecular Packing and Arrangement Govern the Photo-Oxidative Stability of Organic Photovoltaic Materials. *Chemistry Of Materials,* 02 September 2015, vol. 27 (18), 6345-6353
- **ADACHI Y. et al.** Synthesis of organic photosensitizers containing dithienogermole and thiadiazolo[3,4-c]pyridine units for dye-sensitized solar cells. *Dalton Transactions,* 01 January 2016, vol. 45, 13817-13826
- Microwave assisted synthesis of bithiophene based donor-acceptor-donor oligomers and their optoelectronic performances. **CHINNA B. et al.** Journal Of Molecular Structure. Elsevier, 08 March 2017, vol. 1139, 125-129
- **TANG T. et al.** Regiospecific protonation of organic chromophores. *Physical Chemistry Chemical Physics,* 01 January 2016, vol. 18, 18758-18766
- **LECLERC M.** *Journal of the American Chemical Society,* 2008, vol. 130 (2), 732-742
- **JEN A. K.-Y.** *Journal of Materials Chemistry,* 2011, vol. 21, 13247-13255
- **WELCH G. C.** *Journal of the American Chemical Society,* 2011, vol. 133 (12), 4632-4644